# EUROPEAN PATENT APPLICATION

(11) **EP 2 708 605 A1**
(43) Date of publication of application: **19.03.2014**
(21) Application number: 12006529.7
(22) Date of filing: 17.09.2012
(51) Int. Cl.: C12Q 1/68

(54) **ANKRD26 as a marker for diagnosis of thrombocytopenias**

(71) Applicant: Institut Gustave Roussy (IGR), 94805 Villejuif Cedex (FR)
(72) Inventor: Bluteau, Dominique, 75012 Paris (FR); Favier, Rémi, 92330 Sceaux (FR); Raslova, Hana, 91830 Le Coudray Montceaux (FR); Vainchenker, William, 75005 Paris (FR)
(74) Representative: Barbot, Willy

(57) **Abstract**

The present invention relates to an *in vitro* method for diagnosing thrombocytopenia in a subject, said method comprising the step of assessing the ANKRD26 platelet expression in a biological sample from said subject, and wherein a ANKRD26 platelet expression is indicative of a thrombocytopenia.

The present invention also provides a kit for diagnosing a thrombocytopenia in a subject, which comprises at least one ANKRD26 nucleic acid probe or oligonucleotide or antibody for assessing the ANKRD26 platelet expression in a biological sample from said subject.

SEQUENCE LISTING

<110>  Institut Gustave Roussy

<120>  ANKRD26 AS A MARKER FOR DIAGNOSIS OF THROMBOCYTOPENIAS

<130>  IGR-B-0005

<160>  10

<170>  PatentIn version 3.5

<210>  1
<211>  1709
<212>  PRT
<213>  Homo sapiens

<400>  1

Met Lys Lys Ile Phe Ser Lys Lys Gly Glu Ser Pro Leu Gly Ser Phe
1               5                   10                  15

Ala Arg Arg Gln Arg Ser Ser Ala Gly Gly Gly Gly Glu Pro Gly Glu
            20                  25                  30

Gly Ala Tyr Ser Gln Pro Gly Tyr His Val Arg Asp Arg Asp Leu Gly
        35                  40                  45

Lys Ile His Lys Ala Ala Ser Ala Gly Asn Val Ala Lys Val Gln Gln
    50                  55                  60

Ile Leu Leu Leu Arg Lys Asn Gly Leu Asn Asp Arg Asp Lys Met Asn
65                  70                  75                  80

Arg Thr Ala Leu His Leu Ala Cys Ala Asn Gly His Pro Glu Val Val
                85                  90                  95

Thr Leu Leu Val Asp Arg Lys Cys Gln Leu Asn Val Cys Asp Asn Glu
            100                 105                 110

Asn Arg Thr Ala Leu Met Lys Ala Val Gln Cys Gln Glu Glu Lys Cys
        115                 120                 125

Ala Thr Ile Leu Leu Glu His Gly Ala Asp Pro Asn Leu Ala Asp Val
    130                 135                 140

His Gly Asn Thr Ala Leu His Tyr Ala Val Tyr Asn Glu Asp Ile Ser
145                 150                 155                 160

Val Ala Thr Lys Leu Leu Leu Tyr Asp Ala Asn Ile Glu Ala Lys Asn
                165                 170                 175

Lys Asp Asp Leu Thr Pro Leu Leu Leu Ala Val Ser Gly Lys Lys Gln
            180                 185                 190

Gln Met Val Glu Phe Leu Ile Lys Lys Lys Ala Asn Val Asn Ala Val

        195                 200                 205

Asp Lys Leu Glu Ser Ser His Gln Leu Ile Ser Glu Tyr Lys Glu Glu
    210                 215                 220

Arg Ile Pro Lys His Ser Ser Gln Asn Ser Asn Ser Val Asp Glu Ser
225                 230                 235                 240

Ser Glu Asp Ser Leu Ser Arg Leu Ser Gly Lys Pro Gly Val Asp Asp
                245                 250                 255

Ser Trp Pro Thr Ser Asp Asp Glu Asp Leu Asn Phe Asp Thr Lys Asn
            260                 265                 270

Val Pro Lys Pro Ser Leu Ala Lys Leu Met Thr Ala Ser Gln Gln Ser
        275                 280                 285

Arg Lys Asn Leu Glu Ala Thr Tyr Gly Thr Val Arg Thr Gly Asn Arg
    290                 295                 300

Thr Leu Phe Glu Asp Arg Asp Ser Asp Ser Gln Asp Glu Val Val Val
305                 310                 315                 320

Glu Ser Leu Pro Thr Thr Ser Ile Lys Val Gln Cys Phe Ser His Pro
                325                 330                 335

Thr Tyr Gln Ser Pro Asp Leu Leu Pro Lys Pro Ser His Lys Ser Leu
            340                 345                 350

Ala Asn Pro Gly Leu Met Lys Glu Glu Pro Thr Lys Pro Gly Ile Ala
        355                 360                 365

Lys Lys Glu Asn Gly Ile Asp Ile Ile Glu Ser Ala Pro Leu Glu Gln
    370                 375                 380

Thr Asn Asn Asp Asn Leu Thr Tyr Val Asp Glu Val His Lys Asn Asn
385                 390                 395                 400

Arg Ser Asp Met Met Ser Ala Leu Gly Leu Gly Gln Glu Glu Asp Ile
                405                 410                 415

Glu Ser Pro Trp Asp Ser Glu Ser Ile Ser Glu Asn Phe Pro Gln Lys
            420                 425                 430

Tyr Val Asp Pro Leu Ala Gly Ala Ala Asp Gly Lys Glu Lys Asn Ile
        435                 440                 445

Gly Asn Glu Gln Ala Glu Asp Val Phe Tyr Ile Pro Ser Cys Met Ser
    450                 455                 460

Gly Ser Arg Asn Phe Lys Met Ala Lys Leu Glu Asp Thr Arg Asn Val

465                 470                 475                 480

Gly Met Pro Val Ala His Met Glu Ser Pro Glu Arg Tyr Leu His Leu
                485                 490                 495

Lys Pro Thr Ile Glu Met Lys Asp Ser Val Pro Asn Lys Ala Gly Gly
            500                 505                 510

Met Lys Asp Val Gln Thr Ser Lys Ala Ala Glu His Asp Leu Glu Val
        515                 520                 525

Ala Ser Glu Glu Glu Gln Glu Arg Glu Gly Ser Glu Asn Asn Gln Pro
    530                 535                 540

Gln Val Glu Glu Glu Arg Lys Lys His Arg Asn Asn Glu Met Glu Val
545                 550                 555                 560

Ser Ala Asn Ile His Asp Gly Ala Thr Asp Asp Ala Glu Asp Asp Asp
                565                 570                 575

Asp Asp Asp Gly Leu Ile Gln Lys Arg Lys Ser Gly Glu Thr Asp His
            580                 585                 590

Gln Gln Phe Pro Arg Lys Glu Asn Lys Glu Tyr Ala Ser Gly Pro Ala
        595                 600                 605

Leu Gln Met Lys Glu Val Lys Ser Thr Glu Lys Glu Lys Arg Thr Ser
    610                 615                 620

Lys Glu Ser Val Asn Ser Pro Val Phe Gly Lys Ala Ser Leu Leu Thr
625                 630                 635                 640

Gly Gly Leu Leu Gln Val Asp Asp Asp Ser Ser Leu Ser Glu Ile Asp
                645                 650                 655

Glu Asp Glu Gly Arg Pro Thr Lys Lys Thr Ser Asn Glu Lys Asn Lys
            660                 665                 670

Val Lys Asn Gln Ile Gln Ser Met Asp Asp Val Asp Asp Leu Thr Gln
        675                 680                 685

Ser Ser Glu Thr Ala Ser Glu Asp Cys Glu Leu Pro His Ser Ser Tyr
    690                 695                 700

Lys Asn Phe Met Leu Leu Ile Glu Gln Leu Gly Met Glu Cys Lys Asp
705                 710                 715                 720

Ser Val Ser Leu Leu Lys Ile Gln Asp Ala Ala Leu Ser Cys Glu Arg
                725                 730                 735

Leu Leu Glu Leu Lys Lys Asn His Cys Glu Leu Leu Thr Val Lys Ile

            740                 745                 750

Lys Lys Met Glu Asp Lys Val Asn Val Leu Gln Arg Glu Leu Ser Glu
        755                 760                 765

Thr Lys Glu Ile Lys Ser Gln Leu Glu His Gln Lys Val Glu Trp Glu
    770                 775                 780

Arg Glu Leu Cys Ser Leu Arg Phe Ser Leu Asn Gln Glu Glu Glu Lys
785                 790                 795                 800

Arg Arg Asn Ala Asp Thr Leu Tyr Glu Lys Ile Arg Glu Gln Leu Arg
                805                 810                 815

Arg Lys Glu Glu Gln Tyr Arg Lys Glu Val Glu Val Lys Gln Gln Leu
            820                 825                 830

Glu Leu Ser Leu Gln Thr Leu Glu Met Glu Leu Arg Thr Val Lys Ser
        835                 840                 845

Asn Leu Asn Gln Val Val Gln Glu Arg Asn Asp Ala Gln Arg Gln Leu
    850                 855                 860

Ser Arg Glu Gln Asn Ala Arg Met Leu Gln Asp Gly Ile Leu Thr Asn
865                 870                 875                 880

His Leu Ser Lys Gln Lys Glu Ile Glu Met Ala Gln Lys Lys Met Asn
                885                 890                 895

Ser Glu Asn Ser His Ser His Glu Glu Glu Lys Asp Leu Ser His Lys
            900                 905                 910

Asn Ser Met Leu Gln Glu Glu Ile Ala Met Leu Arg Leu Glu Ile Asp
        915                 920                 925

Thr Ile Lys Asn Gln Asn Gln Glu Lys Glu Lys Lys Cys Phe Glu Asp
    930                 935                 940

Leu Lys Ile Val Lys Glu Lys Asn Glu Asp Leu Gln Lys Thr Ile Lys
945                 950                 955                 960

Gln Asn Glu Glu Thr Leu Thr Gln Thr Ile Ser Gln Tyr Asn Gly Arg
                965                 970                 975

Leu Ser Val Leu Thr Ala Glu Asn Ala Met Leu Asn Ser Lys Leu Glu
            980                 985                 990

Asn Glu Lys Gln Ser Lys Glu Arg Leu Glu Ala Glu Val Glu Ser Tyr
        995                 1000                1005

His Ser Arg Leu Ala Ala Ala Ile His Asp Arg Asp Gln Ser Glu

1010                    1015                    1020

Thr Ser  Lys Arg Glu Leu Glu  Leu Ala Phe Gln Arg  Ala Arg Asp
    1025                    1030                    1035

Glu Cys  Ser Arg Leu Gln Asp  Lys Met Asn Phe Asp  Val Ser Asn
    1040                    1045                    1050

Leu Lys  Asp Asn Asn Glu Ile  Leu Ser Gln Gln Leu  Phe Lys Thr
    1055                    1060                    1065

Glu Ser  Lys Leu Asn Ser Leu  Glu Ile Glu Phe His  His Thr Arg
    1070                    1075                    1080

Asp Ala  Leu Arg Glu Lys Thr  Leu Gly Leu Glu Arg  Val Gln Lys
    1085                    1090                    1095

Asp Leu  Ser Gln Thr Gln Cys  Gln Met Lys Glu Met  Glu Gln Lys
    1100                    1105                    1110

Tyr Gln  Asn Glu Gln Val Lys  Val Asn Lys Tyr Ile  Gly Lys Gln
    1115                    1120                    1125

Glu Ser  Val Glu Glu Arg Leu  Ser Gln Leu Gln Ser  Glu Asn Met
    1130                    1135                    1140

Leu Leu  Arg Gln Gln Leu Asp  Asp Ala His Asn Lys  Ala Asp Asn
    1145                    1150                    1155

Lys Glu  Lys Thr Val Ile Asn  Ile Gln Asp Gln Phe  His Ala Ile
    1160                    1165                    1170

Val Gln  Lys Leu Gln Ala Glu  Ser Glu Lys Gln Ser  Leu Leu Leu
    1175                    1180                    1185

Glu Glu  Arg Asn Lys Glu Leu  Ile Ser Glu Cys Asn  His Leu Lys
    1190                    1195                    1200

Glu Arg  Gln Tyr Gln Tyr Glu  Asn Glu Lys Ala Glu  Arg Glu Val
    1205                    1210                    1215

Val Val  Arg Gln Leu Gln Gln  Glu Leu Ala Asp Thr  Leu Lys Lys
    1220                    1225                    1230

Gln Ser  Met Ser Glu Ala Ser  Leu Glu Val Thr Ser  Arg Tyr Arg
    1235                    1240                    1245

Ile Asn  Leu Glu Asp Glu Thr  Gln Asp Leu Lys Lys  Lys Leu Gly
    1250                    1255                    1260

Gln Ile  Arg Asn Gln Leu Gln  Glu Ala Gln Asp Arg  His Thr Glu

1265                1270                1275

Ala Val  Arg Cys Ala Glu Lys  Met Gln Asp His Lys  Gln Lys Leu
    1280                1285                1290

Glu Lys  Asp Asn Ala Lys Leu  Lys Val Thr Val Lys  Lys Gln Met
    1295                1300                1305

Asp Lys  Ile Glu Glu Leu Gln  Lys Asn Leu Leu Asn  Ala Asn Leu
    1310                1315                1320

Ser Glu  Asp Glu Lys Glu Gln  Leu Lys Lys Leu Met  Glu Leu Lys
    1325                1330                1335

Gln Ser  Leu Glu Cys Asn Leu  Asp Gln Glu Met Lys  Lys Asn Val
    1340                1345                1350

Glu Leu  Glu Arg Glu Ile Thr  Gly Phe Lys Asn Leu  Leu Lys Met
    1355                1360                1365

Thr Arg  Lys Lys Leu Asn Glu  Tyr Glu Asn Gly Glu  Phe Ser Phe
    1370                1375                1380

His Gly  Asp Leu Lys Thr Ser  Gln Phe Glu Met Asp  Ile Gln Ile
    1385                1390                1395

Asn Lys  Leu Lys His Lys Ile  Asp Asp Leu Thr Ala  Glu Leu Glu
    1400                1405                1410

Thr Ala  Gly Ser Lys Cys Leu  His Leu Asp Thr Lys  Asn Gln Ile
    1415                1420                1425

Leu Gln  Glu Glu Leu Leu Ser  Met Lys Thr Val Gln  Lys Lys Cys
    1430                1435                1440

Glu Lys  Leu Gln Lys Asn Lys  Lys Lys Leu Glu Gln  Glu Val Ile
    1445                1450                1455

Asn Leu  Arg Ser His Ile Glu  Arg Asn Met Val Glu  Leu Gly Gln
    1460                1465                1470

Val Lys  Gln Tyr Lys Gln Glu  Ile Glu Glu Arg Ala  Arg Gln Glu
    1475                1480                1485

Ile Ala  Glu Lys Leu Lys Glu  Val Asn Leu Phe Leu  Gln Ala Gln
    1490                1495                1500

Ala Ala  Ser Gln Glu Asn Leu  Glu Gln Phe Arg Glu  Asn Asn Phe
    1505                1510                1515

Ala Ser  Met Lys Ser Gln Met  Glu Leu Arg Ile Lys  Asp Leu Glu

1520                    1525                    1530

Ser Glu Leu Ser Lys Ile Lys Thr Ser Gln Glu Asp Phe Asn Lys
    1535                    1540                    1545

Thr Glu Leu Glu Lys Tyr Lys Gln Leu Tyr Leu Glu Glu Leu Lys
    1550                    1555                    1560

Val Arg Lys Ser Leu Ser Ser Lys Leu Thr Lys Thr Asn Glu Arg
    1565                    1570                    1575

Leu Ala Glu Val Asn Thr Lys Leu Leu Val Glu Lys Gln Gln Ser
    1580                    1585                    1590

Arg Ser Leu Phe Thr Thr Leu Thr Thr Arg Pro Val Met Glu Pro
    1595                    1600                    1605

Pro Cys Val Gly Asn Leu Asn Asn Ser Leu Asp Leu Asn Arg Lys
    1610                    1615                    1620

Leu Ile Pro Arg Glu Asn Leu Val Ile Ser Thr Ser Asn Pro Arg
    1625                    1630                    1635

Ala Ser Asn Asn Ser Met Glu Asn Tyr Leu Ser Lys Met Gln Gln
    1640                    1645                    1650

Glu Leu Glu Lys Asn Ile Thr Arg Glu Leu Lys Glu Ala Ala Ala
    1655                    1660                    1665

Glu Leu Glu Ser Gly Ser Ile Ala Ser Pro Leu Gly Ser Thr Asp
    1670                    1675                    1680

Glu Ser Asn Leu Asn Gln Asp Leu Val Trp Lys Ala Ser Arg Glu
    1685                    1690                    1695

Tyr Val Gln Val Leu Lys Lys Asn Tyr Met Ile
    1700                    1705

<210>  2
<211>  6776
<212>  DNA
<213>  homo sapiens

<400>  2
acttgtgtct agcgggatcg cttgcttggt aacccggagg gagagattgg aaaccgcgga      60

gtttcctttg ggaggctgcg gccagccggg gctgacttgt tatgttgggc tccggaggcc     120

gttaagagcc gagagagaca tgaggtgtct ctgaagcccg gtcgcctggg ccatgaagaa     180

gatttttagt aagaagggcg agtcgccctt gggctccttc gcgcggcggc agaggagcag     240

cgcgggaggc gggggcgagc cgggggaggg cgcctactcg cagcccggct accacgtccg     300

agaccgagat ctcggcaaga tccacaaagc tgccagcgcg ggtaatgtgg cgaaagtgca     360

gcagatcctt ttgctcagga agaatggctt gaacgataga gacaagatga acaggacggc 420
tctacatttg gcctgtgcca atggtcatcc agaagtagta actctcctgg tggacagaaa 480
atgccagctc aatgtctgtg acaacgaaaa caggacagct ctgatgaagg ctgtacaatg 540
ccaggaagag aaatgtgcaa ctattctgct agaacatggt gctgatccaa atcttgcgga 600
tgtccatggc aacactgctc ttcactatgc tgtctataat gaggacatat cagtagcaac 660
aaagctgctt ttgtatgatg caaatattga agcaaaaaac aaggatgacc tcacaccact 720
tttacttgca gtaagtggaa aaaagcagca aatggtggaa tttttaataa agaaaaaagc 780
aaatgtaaat gcagtagata agttggaaag cagtcaccaa ctaatttcag aatataaaga 840
agaaaggata cctaaacatt cttctcaaaa tagtaattca gtggatgaaa gctctgaaga 900
ctccttaagc aggctttctg gcaaaccggg tgttgatgat tcatggccta cctcagatga 960
cgaagacctc aattttgata ctaagaatgt cccaaaacca agcttagcaa agctaatgac 1020
tgcttctcag caatccagga aaaatttaga agcaacatat ggcactgtga gaacaggaaa 1080
tagaactttg tttgaggata gagattccga tagtcaagat gaagttgtgg ttgaaagcct 1140
tcctacaaca tcaatcaaag tccagtgctt ttctcatcct acctatcaat cacctgacct 1200
tcttccaaaa ccttcccaca agtcgttagc aaaccctggt cttatgaagg aagaaccaac 1260
aaagccaggc attgcaaaaa aagaaaatgg tattgatatt attgaaagtg ctccactaga 1320
gcaaacaaat aatgacaatt tgacttatgt tgatgaagtg cacaaaaata atagaagtga 1380
tatgatgtcc gcattaggat taggacaaga ggaagatata gaatcacctt gggattctga 1440
gagtatctct gagaattttc cacagaagta tgttgatcct ttagctgggg ctgcagacgg 1500
aaaagaaaaa aatataggaa atgaacaagc agaagatgtg ttttatatac cttcttgcat 1560
gagtggatca agaaacttta agatggctaa actagaggat acaagaaatg taggcatgcc 1620
agtagcccac atggagtctc ctgagagata tcttcacttg aagcctacca ttgaaatgaa 1680
agattctgtt ccaaataaag caggaggaat gaaggatgta caaacatcca aagcagctga 1740
acatgactta gaagtagcat cagaagaaga gcaagaaagg gaagggagtg aaaataacca 1800
gccacaggtt gaagaagaaa ggaaaaaaca cagaaataat gaaatggaag tatcagcaaa 1860
catacatgat ggtgctactg atgatgctga agatgatgat gatgatgatg gattaattca 1920
aaaaagaaag agtggagaaa ctgatcatca gcaatttccc aggaaggaaa ataaagagta 1980
tgctagtggt cctgccttgc aaatgaagga agtaaagagc actgaaaaag aaaaacggac 2040
ctcgaaagaa tctgtgaatt caccagtgtt tgggaaggcc agtttactaa ctggtggcct 2100
gctacaagtg gatgatgaca gcagtttaag tgaaatagat gaggatgaag gaaggcctac 2160
taagaaaaca tctaatgaaa agaacaaggt caaaaaccaa atacagtcta tggatgatgt 2220
tgatgactta actcagtcat ctgaaacagc ctcagaggat tgtgagctac cccactctag 2280
ttacaagaat tttatgttgc tcattgaaca acttggaatg gagtgtaaag attctgttag 2340
cctattgaaa atccaggatg cagctctttc atgtgaaaga ttattagaac ttaaaaaaaa 2400

tcactgtgaa ctacttacag taaaaattaa aaaaatggaa gacaaggtta atgtactaca 2460
aagggagcta tctgaaacaa aagaaataaa atcacagtta gagcatcaaa aagttgaatg 2520
ggaacgagaa ctgtgctctt tgagatttag cttaaaccaa gaagaagaga agagaagaaa 2580
tgctgatacg ttgtatgaaa aaattaggga acagttaaga agaaaagaag agcaatatag 2640
gaaagaagtt gaagtgaaac aacagcttga actgagtctc caaacactgg agatggaatt 2700
gaggactgta aaaagtaatt tgaatcaggt cgttcaagag cgaaatgacg ctcagaggca 2760
actttctcga gaacagaatg ccagaatgtt acaagatgga attctgacca atcacctttc 2820
caaacaaaag gagattgaaa tggctcaaaa gaaaatgaat tctgagaatt ctcatagtca 2880
tgaagaagaa aaagacctat cgcataaaaa tagcatgttg caggaagaaa ttgctatgct 2940
aagactagaa atagacacaa taaaaaatca aaaccaggaa aaagaaaaga aatgttttga 3000
ggaccttaaa attgtaaaag aaaagaatga agaccttcag aagactataa aacagaatga 3060
ggaaacatta acacaaacaa tatcccagta taatggacgg cttagtgttc tgacagctga 3120
gaatgcaatg ctaaattcta aactggagaa tgaaaagcaa agcaaggaaa gactggaagc 3180
agaagttgaa tcataccatt ctagattggc tgctgctata catgatcgtg atcaaagtga 3240
gacatcaaaa agagaactag aacttgcttt ccagagagca agagatgaat gttctcgttt 3300
acaggacaaa atgaattttg atgtgtctaa cctaaaagat aacaatgaga ttctttctca 3360
acaactattt aaaactgaaa gtaaactcaa tagcctagaa attgagttcc atcacacgag 3420
agatgccctc agagaaaaga ctttgggttt agaacgggta caaaaggacc taagccaaac 3480
acagtgtcaa atgaaggaaa tggaacaaaa gtatcaaaat gaacaagtta aagtgaataa 3540
atacattgga aagcaggagt ctgtagagga gagattgtct caactacaaa gtgagaatat 3600
gttgcttcga caacaactgg atgatgccca caacaaggct gacaataaag agaagacagt 3660
gattaatatc caagaccagt ttcatgctat tgtgcaaaaa cttcaagctg agagtgaaaa 3720
gcaaagtctt ctgctagaag aaagaaataa ggagttaatc agtgaatgta atcacttaaa 3780
agaaagacag tatcaatatg aaaatgaaaa ggcagaaaga gaagttgttg tgagacaact 3840
tcaacaagaa ctagctgata ccctaaaaaa acaatctatg tcagaggctt cactggaggt 3900
tacgtcacgt tatcgtatta atttagaaga tgagacacag gatttaaaga agaaattagg 3960
tcaaatcaga aatcaattgc aagaagcaca ggatcgacat acagaagctg tcagatgtgc 4020
tgagaagatg caagatcaca agcaaaagct tgaaaaagat aatgccaagt taaaagttac 4080
agtcaaaaag caaatggaca aaattgagga gcttcagaaa aacctgttaa atgcaaattt 4140
gtctgaagat gaaaaggaac aattaaagaa acttatggaa ttaaaacagt cactggaatg 4200
taatttggat caagaaatga agaaaaatgt tgaattagaa agagagataa ctggatttaa 4260
gaacctctta aaaatgacaa gaaagaagtt aaatgaatat gaaaatggag aatttagttt 4320
ccatggagat ttaaaaacta gtcaatttga aatggatatt cagattaata agctaaaaca 4380
taagattgat gatcttacag cagaactgga gactgcaggt tcaaaatgtc tacatctgga 4440

tacaaagaac caaattcttc aagaggagtt gttatctatg aaaacagtac aaaagaaatg 4500
tgaaaaacta cagaagaata aaaagaagtt ggaacaagaa gtgatcaacc tgagaagtca 4560
tatagaaagg aatatggtag aacttggtca agtcaaacag tataaacagg agattgaaga 4620
aagagcaaga caggaaatag cagaaaaatt aaaagaagtc aatctatttt tacaggcaca 4680
agcagcatct caagaaaact tagagcagtt tagagagaat aattttgctt caatgaaaag 4740
tcagatggaa ctcagaatta aagatctgga atctgaactc tccaaaataa aaacttctca 4800
agaagacttt aataaaaccg aactggaaaa atataagcaa ctctatctag aagaattaaa 4860
agttagaaaa tctttgtcaa gtaaactaac caaaactaat gagaggctag cagaggtcaa 4920
caccaaactt cttgtggaaa aacagcagag cagatctttg ttcaccactc tcactaccag 4980
gccagtcatg gagccacctt gtgtgggaaa tcttaataat agtttagatc tcaacagaaa 5040
acttattcca agagaaaact tagtgatctc tacctcaaat ccacgggctt caaataatag 5100
catggagaac tacttgagca agatgcagca ggagttggaa aaaaatataa ctagagaact 5160
caaagaagct gctgctgaat tggaatctgg atcaatagct tcccctctag ggtctactga 5220
tgagtcaaat ctaaatcaag atctagtttg gaaagcatca agagaatatg tacaggtttt 5280
aaagaaaaat tatatgatct gaaagataag attttattac tgggctgttt atgtgacatt 5340
ttgtttctca ttaaatatta atatatgaca tgtaaaaata ttaaaggaac gtatttttgt 5400
atcatatatg tcagatcaaa atttatatag tattttaaac aatgttactt ggcttcttta 5460
acttttaagt ggattttgcc aatgaaaacc aggaatattg agttttacat actcaaactg 5520
cccaaatgtg agctgtttaa acagccaaac cactaagttg tcattaatac tttagtgcag 5580
ttaattgatg gcttatttgt attttgtaat ttttatcact atatgtagtg gtagatgtct 5640
aattggactt agacatcatt ttgttgtgca ggaactgtgg tcattgttca tgtttggatg 5700
tattacaatt gttacagtgc catcaaattt acataatttt aattttaagc actgatttat 5760
atagctgttc tcagggagaa acaagatttg cctgattcac cttttctgtt tgtttattta 5820
tttttaatta accttttgga aatagccttc agtttagaga aaatttcgaa gttttagtac 5880
aaagactttc tcctccgaac aatttgagag taaattgctg acattatgtc atcacccctg 5940
attactttag tgggcattcc tacaagtcag gacattctac ataactacag tgcaaacctt 6000
gcaatcagaa agtcaacatt aatacattct agtttgttac ttctctgtaa tgtcctttat 6060
agcaaaagga tcccattcta atccatggct tgtatttagt tgttgtatct gttagatctt 6120
tcagtctgga acagttcctc agtctttcct tgaccttcat gaccttgaat attttgaaga 6180
gtaaagtctg gtaatataag aatgtccctc aattgggttt catggtgatt agatttagat 6240
tatatgattt tttggcagga gtatcacaga agtgacccag tgatcttctc attgcatctt 6300
acctgtgacc ctacagtttt gatttgccct gttactggta atgttagcat tgatcactaa 6360
cagtggtatc tgctgaattt ctcctctgta atgttatttt ttcaccttgt ggttaattag 6420
tattttacag ggaggtaatt tgagactatg tacatatctc tactccccat cgaaccctca 6480

atttattcat ttgcttgtat caaataaacc atggattcat ggtttatttt attcagtgag 6540

ttataattca ttaatattgt tattaaaaat ttgtttttct tttaagaaat gttatatttt 6600

gacaacttca gacttacaga aagattataa gaggtagtac aaataatttt tggatattat 6660

tccccaaatg ttaacatttt actgcattta ctttatcctt tctccccctt ctcctcctgt 6720

ctttctagat gaatatgaat ataggtactt aatacagatt ttttttctaa actgtt 6776

<210> 3
<211> 1976
<212> PRT
<213> homo sapiens

<400> 3

<210>  4
<211>  7685
<212>  DNA
<213>  homo sapiens

<400>  4
agcagtgcta aaggagcccg gcggaggcag cggtgggttt gggactgagg cgctggatct      60

gtggtcgcgg ctggggacgt gcgcccgcgc caccatcttc ggctgaagag gcaattgctt     120

ttggatcgtt ccatttacaa tggcgcagag aactggactc gaggatccag agaggtatct     180

ctttgtggac agggctgtca tctacaaccc tgccactcaa gctgattgga cagctaaaaa     240

gctagtgtgg attccatcag aacgccatgg ttttgaggca gctagtatca aagaagaacg 300
gggagatgaa gttatggtgg agttggcaga gaatggaaag aaagcaatgg tcaacaaaga 360
tgatattcag aagatgaacc cacctaagtt ttccaaggtg gaggatatgg cagaattgac 420
atgcttgaat gaagcttccg ttttacataa tctgaaggat cgctactatt caggactaat 480
ctatacttat tctggactct tctgtgtagt tataaaccct tacaagaatc ttccaattta 540
ctctgagaat attattgaaa tgtacagagg gaagaagcgt catgagatgc ctccacacat 600
ctatgctata tctgaatctg cttacagatg catgcttcaa gatcgtgagg accagtcaat 660
tctttgcacg ggtgagtcag gtgctgggaa gacagaaaat acaaagaaag ttattcagta 720
ccttgcccat gttgcttctt cacataaagg aagaaaggac cataatattc ctggggaact 780
tgaacggcag cttttgcaag caaatccaat tctggaatca tttggaaatg cgaagactgt 840
gaaaaatgat aactcatctc gttttggcaa atttattcgg atcaactttg atgtaactgg 900
ctatatcgtt ggggccaaca ttgaaacata ccttctggaa aagtctcgtg ctgttcgtca 960
agcaaaagat gaacgtactt ttcatatctt ttaccagttg ttatctggag caggagaaca 1020
cctaaagtct gatttgcttc ttgaaggatt taataactac aggtttctct ccaatggcta 1080
tattcctatt ccgggacagc aagacaaaga taatttccag gagaccatgg aagcaatgca 1140
cataatgggc ttctcccatg aagagattct gtcaatgctt aaagtagtat cttcagtgct 1200
acagtttgga aatatttctt tcaaaaagga gagaaatact gatcaagctt ccatgccaga 1260
aaatacagtt gcgcagaagc tctgccatct tcttgggatg aatgtgatgg agtttactcg 1320
ggccatcctg actccccgga tcaaggtcgg ccgagactat gtgcaaaaag cccagaccaa 1380
agaacaggca gattttgcag tagaagcatt ggcaaaagct acctatgagc ggctctttcg 1440
ctggctcgtt catcgcatca ataaagctct ggataggacc aaacgtcagg gagcatcttt 1500
cattggaatc ctggatattg ctggatttga aatttttgag ctgaactcct ttgaacaact 1560
ttgcatcaac tacaccaatg agaagctgca gcagctgttc aaccacacca tgtttatcct 1620
agaacaagag gaataccagc gcgaaggcat cgagtggaac ttcatcgatt tcgggctgga 1680
tctgcagcca tgcatcgacc taatagagag acctgcgaac cctcctggtg tactggccct 1740
tttggatgaa gaatgctggt tccctaaagc cacagataaa acctttgttg aaaaactggt 1800
tcaagagcaa ggttcccact ccaagtttca gaaacctcga caattaaaag acaaagctga 1860
tttttgcatt atacattatg cagggaaggt ggactataag gcagatgagt ggctgatgaa 1920
gaatatggac cccctgaatg acaacgtggc cacccttttg caccagtcat cagacagatt 1980
tgtggcagag ctttggaaag atgtggaccg tatcgtgggt ctggatcaag tcactggtat 2040
gactgagaca gcttttggct ccgcatataa aaccaagaag ggcatgtttc gtaccgttgg 2100
gcaactctac aaagaatctc tcaccaagct gatggcaact ctccgaaaca ccaaccctaa 2160
ctttgttcgt tgtatcattc caaatcacga gaagagggct ggaaaattgg atccacacct 2220
agtcctagat cagcttcgct gtaatggtgt cctggaaggg atccgaatct gtcgccaggg 2280

cttccctaac cgaatagttt tccaggaatt cagacagaga tatgagatcc taactccaaa 2340
tgctattcct aaaggtttta tggatggtaa acaggcctgt gaacgaatga tccgggcttt 2400
agaattggac ccaaacttgt acagaattgg acagagcaag atatttttca gagctggagt 2460
tctggcacac ttagaggaag aaagagattt aaaaatcacc gatatcatta tcttcttcca 2520
ggccgtttgc agaggttacc tggccagaaa ggcctttgcc aagaagcagc agcaactaag 2580
tgccttaaag gtcttgcagc ggaactgtgc cgcgtacctg aaattacggc actggcagtg 2640
gtggcgagtc ttcacaaagg tgaagccgct tctacaagtg actcgccagg aggaagaact 2700
tcaggccaaa gatgaagagc tgttgaaggt gaaggagaag cagacgaagg tggaaggaga 2760
gctggaggag atggagcgga agcaccagca gcttttagaa gagaagaata tccttgcaga 2820
acaactacaa gcagagactg agctctttgc tgaagcagaa gagatgaggg caagacttgc 2880
tgctaaaaag caggaattag aagagattct acatgacttg gagtctaggg ttgaagaaga 2940
agaagaaaga aaccaaatcc tccaaaatga aaagaaaaaa atgcaagcac atattcagga 3000
cctggaagaa cagctagacg aggaggaagg ggctcggcaa aagctgcagc tggaaaaggt 3060
gacagcagag gccaagatca agaagatgga agaggagatt ctgcttctcg aggaccaaaa 3120
ttccaagttc atcaaagaaa agaaactcat ggaagatcgc attgctgagt gttcctctca 3180
gctggctgaa gaggaagaaa aggcgaaaaa cttggccaaa atcaggaata agcaagaagt 3240
gatgatctca gatttagaag aacgcttaaa gaaggaagaa aagactcgtc aggaactgga 3300
aaaggccaaa agaaaactcg acggggagac gaccgacctg caggaccaga tcgcagagct 3360
gcaggcgcag attgatgagc tcaagctgca gctggccaag aaggaggagg agctgcaggg 3420
cgcactggcc agaggtgatg atgaaacact ccataagaac aatgccctta aagttgtgcg 3480
agagctacaa gcccaaattg ctgaacttca ggaagacttt gaatccgaga aggcttcacg 3540
gaacaaggcc gaaaagcaga aaagggactt gagtgaggaa ctggaagctc tgaaaacaga 3600
gctggaggac acgctggaca ccacggcagc ccagcaggaa ctacgtacaa aacgtgaaca 3660
agaagtggca gagctgaaga aagctcttga ggaggaaact aagaaccatg aagctcaaat 3720
ccaggacatg agacaaagac acgcaacagc cctggaggag ctctcagagc agctggaaca 3780
ggccaagcgg ttcaaagcaa atctagagaa gaacaagcag ggcctggaga cagataacaa 3840
ggagctggcg tgtgaggtga aggtcctgca gcaggtcaag gctgagtctg agcacaagag 3900
gaagaagctc gacgcgcagg tccaggagct ccatgccaag gtctctgaag gcgacaggct 3960
cagggtggag ctggcggaga aagcaagtaa gctgcagaat gagctagata atgtctccac 4020
ccttctggaa gaagcagaga agaagggtat taaatttgct aaggatgcag ctagtcttga 4080
gtctcaacta caggatacac aggagcttct tcaggaggag acacgccaga aactaaacct 4140
gagcagtcgg atccggcagc tggaagagga gaagaacagt cttcaggagc agcaggagga 4200
ggaggaggag gccaggaaga acctggagaa gcaagtgctg gccctgcagt cccagttggc 4260
tgataccaag aagaaagtag atgacgacct gggaacaatt gaaagtctgg aagaagccaa 4320

gaagaagctt ctgaaggacg cggaggccct gagccagcgc ctggaggaga aggcactggc 4380
gtatgacaaa ctggagaaga ccaagaaccg cctgcagcag gagctggacg acctcacggt 4440
ggacctggac caccagcgcc aggtcgcctc caacttggag aagaagcaga agaagtttga 4500
ccagctgtta gcagaagaga agagcatctc tgctcgctat gccgaagagc gggaccgggc 4560
cgaagccgag gccagagaga aagaaaccaa agccctgtca ctggcccggg ccctcgagga 4620
agccctggag gccaaggagg agtttgagag gcagaacaag cagctccgag cagacatgga 4680
agacctcatg agctccaaag atgatgtggg aaaaaacgtt cacgaacttg aaaaatccaa 4740
acgggcccta gagcagcagg tggaggaaat gaggacccag ctggaggagc tggaagacga 4800
actccaggcc acggaagatg ccaagcttcg tctggaggtc aacatgcagg ccatgaaggc 4860
gcagttcgag agagacctgc aaaccaggga tgagcagaat gaagagaaga agcggctgct 4920
gatcaaacag gtgcgggagc tcgaggcgga gctggaggat gagaggaaac agcgggcgct 4980
tgctgtagct tcaaagaaaa agatggagat agacctgaag gacctcgaag cccaaatcga 5040
ggctgcgaac aaagctcggg atgaggtgat taagcagctc cgcaagctcc aggctcagat 5100
gaaggattac caacgtgaat tagaagaagc tcgtgcatcc agagatgaga tttttgctca 5160
atccaaagag agtgaaaaga aattgaagag tctggaagca gaaatccttc aattgcagga 5220
ggaacttgcc tcatctgagc gagcccgccg acacgccgag caggagagag atgagctggc 5280
ggacgagatc accaacagcg cctctggcaa gtccgcgctg ctggatgaga agcggcgtct 5340
ggaagctcgg atcgcacagc tggaggagga gctggaagag gagcagagca acatggagct 5400
gctcaacgac cgcttccgca agaccactct acaggtggac acactgaacg ccgagctagc 5460
agccgagcgc agcgccgccc agaagagtga caatgcacgc cagcaactgg agcggcagaa 5520
caaggagctg aaggccaagc tgcaggaact cgagggtgct gtcaagtcta agttcaaggc 5580
caccatctca gccctggagg ccaagattgg gcagctggag gagcagcttg agcaggaagc 5640
caaggaacga gcagccgcca acaaattagt ccgtcgcact gagaagaagc tgaaagaaat 5700
cttcatgcag gttgaggatg agcgtcgaca cgcggaccag tataaagagc agatggagaa 5760
ggccaacgct cggatgaagc agcttaaacg ccagctggag gaagcagaag aagaagcgac 5820
gcgtgccaac gcatctcggc gtaaactcca gcgggaactg gatgatgcca ccgaggccaa 5880
cgagggcctg agccgcgagg tcagcaccct gaagaaccgg ctgaggcggg gtggccccat 5940
cagcttctct tccagccgat ctggccggcg ccagctgcac cttgaaggag cttccctgga 6000
gctctccgac gatgacacag aaagtaagac cagtgatgtc aacgagacgc agccacccca 6060
gtcagagtaa agttgcagga agccagagga ggcaatacag tgggacagtt aggaatgcac 6120
ccggggcctc ctgcagattt cggaaattgg caagctacgg gattccttcc tgaaagatca 6180
actgtgtctt aaggctctcc agcctatgca tactgtatcc tgcttcagac ttaggtacaa 6240
ttgctcccct ttttatatat agacacacac aggacacata tattaaacag attgtttcat 6300
cattgcatct attttccata tagtcatcaa gagaccattt tataaaacat ggtaagaccc 6360

tttttaaaac aaactccagg cccttggttg cgggtcgctg ggttattggg gcagcgccgt 6420
ggtcgtcact cagtcgctct gcatgctctc tgtcatacag acaggtaacc tagttctgtg 6480
ttcacgtggc ccccgactcc tcagccacat caagtctcct agaccactgt ggactctaaa 6540
ctgcacttgt ctctctcatt tccttcaaat aatgatcaat gctatttcag tgagcaaact 6600
gtgaaagggg ctttggaaag agtaggaggg gtgggctgga tcggaagcaa cacccatttg 6660
gggttaccat gtccatcccc caaggggggc cctgcccctc gagtcgatgg tgtcccgcat 6720
ctactcatgt gaactggcct tggcgagggc tggtctgtgc atagaaggga tagtggccac 6780
actgcagctg aggccccagg tggcagccat ggatcatgta gacttccaga tggtctcccg 6840
aaccgcctgg ctctgccggc gccctcctca cgtcaggagc aagcagccgt ggacccctaa 6900
gccgagctgg tggaaggccc ctccccgtcg ccagccgggc cctcatgctg accttgcaaa 6960
ttcagccgct gctttgagcc caaaatggga atattggttt tgtgtccgag gcttgttcca 7020
agtttgtcaa tgaggtttat ggagcctcca gaacagatgc catcttcctg aatgttgaca 7080
tgccagtggg tgtgactcct tcatttttcc ttctcccttc cctttggaca gtgttacagt 7140
gaacacttag catcctgttt ttggttggta gttaagcaaa ctgacattac ggaaagtgcc 7200
ttagacacta cagtactaag acaatgttga atatatcatt cgcctctata acaatttaat 7260
gtattcagtt ttgactgtgc ttcatatcat gtacctctct agtcaaagtg gtattacaga 7320
cattcagtga caatgaatca gtgttaattc taaatccttg atcctctgca atgtgcttga 7380
aaacacaaac cttttgggtt aaaagcttta acatctatta ggaagaattt gtcctgtggg 7440
tttggaatct tggattttcc ccctttatga actgtactgg ctgttgacca ccagacacct 7500
gaccgcaaat atcttttctt gtattcccat atttctagac aatgattttt gtaagacaat 7560
aaatttattc attatagata tttgcgcctg ctctgtttac ttgaagaaaa aagcacccgt 7620
ggagaataaa gagacctcaa taaacaagaa taatcatgtg aacgtggaaa aaaaaaaaaa 7680
aaaaa 7685

<210> 5
<211> 23
<212> DNA
<213> Artificial Sequence

<220>
<223> primers

<400> 5
ctatgtcaga ggcttcactg gag 23

<210> 6
<211> 23
<212> DNA
<213> Artificial Sequence

<220>
<223> primers

<400>  6
ctcagcacat ctgacagctt ctg                                              23

<210>  7
<211>  22
<212>  DNA
<213>  Artificial Sequence

<220>
<223>  primers

<400>  7
gctgatggca actctccgaa ac                                               22

<210>  8
<211>  22
<212>  DNA
<213>  Artificial Sequence

<220>
<223>  primers

<400>  8
cttccaggac accattacag cg                                               22

<210>  9
<211>  1710
<212>  PRT
<213>  Homo sapiens

<400>  9
   Met Lys Lys Ile Phe Ser Lys Lys Gly Glu Ser Pro Leu Gly Ser Phe
   1               5                   10                  15
   Ala Arg Arg Gln Arg Ser Ser Ala Gly Gly Gly Gly Glu Pro Gly Glu
               20                  25                  30
   Gly Ala Tyr Ser Gln Pro Gly Tyr His Val Arg Asp Arg Asp Leu Gly
           35                  40                  45
   Lys Ile His Lys Ala Ala Ser Ala Gly Asn Val Ala Lys Val Gln Gln
       50                  55                  60
   Ile Leu Leu Leu Arg Lys Asn Gly Leu Asn Asp Arg Asp Lys Met Asn
   65                  70                  75                  80
   Arg Thr Ala Leu His Leu Ala Cys Ala Asn Gly His Pro Glu Val Val
                   85                  90                  95
   Thr Leu Leu Val Asp Arg Lys Cys Gln Leu Asn Val Cys Asp Asn Glu
               100                 105                 110
   Asn Arg Thr Ala Leu Met Lys Ala Val Gln Cys Gln Glu Glu Lys Cys
           115                 120                 125
   Ala Thr Ile Leu Leu Glu His Gly Ala Asp Pro Asn Leu Ala Asp Val
   130                 135                 140
   His Gly Asn Thr Ala Leu His Tyr Ala Val Tyr Asn Glu Asp Ile Ser
   145                 150                 155                 160
   Val Ala Thr Lys Leu Leu Leu Tyr Asp Ala Asn Ile Glu Ala Lys Asn
                   165                 170                 175
   Lys Asp Asp Leu Thr Pro Leu Leu Leu Ala Val Ser Gly Lys Lys Gln
               180                 185                 190
   Gln Met Val Glu Phe Leu Ile Lys Lys Lys Ala Asn Val Asn Ala Val
           195                 200                 205
   Asp Lys Leu Glu Ser Ser His Gln Leu Ile Ser Glu Tyr Lys Glu Glu
       210                 215                 220
   Arg Ile Pro Lys His Ser Ser Gln Asn Ser Asn Ser Val Asp Glu Ser
   225                 230                 235                 240
   Ser Glu Asp Ser Leu Ser Arg Leu Ser Gly Lys Pro Gly Val Asp Asp
                   245                 250                 255
   Ser Trp Pro Thr Ser Asp Asp Glu Asp Leu Asn Phe Asp Thr Lys Asn
               260                 265                 270
   Val Pro Lys Pro Ser Leu Ala Lys Leu Met Thr Ala Ser Gln Gln Ser
           275                 280                 285
   Arg Lys Asn Leu Glu Ala Thr Tyr Gly Thr Val Arg Thr Gly Asn Arg
       290                 295                 300
   Thr Leu Phe Glu Asp Arg Asp Ser Asp Ser Gln Asp Glu Val Val Val
   305                 310                 315                 320
   Glu Ser Leu Pro Thr Thr Ser Ile Lys Val Gln Cys Phe Ser His Pro
                   325                 330                 335
   Thr Tyr Gln Ser Pro Asp Leu Leu Pro Lys Pro Ser His Lys Ser Leu
               340                 345                 350
   Ala Asn Pro Gly Leu Met Lys Glu Glu Pro Thr Lys Pro Gly Ile Ala
           355                 360                 365
   Lys Lys Glu Asn Gly Ile Asp Ile Ile Glu Ser Ala Pro Leu Glu Gln
       370                 375                 380
   Thr Asn Asn Asp Asn Leu Thr Tyr Val Asp Glu Val His Lys Asn Asn
   385                 390                 395                 400
   Arg Ser Asp Met Met Ser Ala Leu Gly Leu Gly Gln Glu Glu Asp Ile
   405 410 415
   Glu Ser Pro Trp Asp Ser Glu Ser Ile Ser Glu Asn Phe Pro Gln Lys
   420 425 430
   Tyr Val Asp Pro Leu Ala Gly Ala Ala Asp Gly Lys Glu Lys Asn Ile
   435 440 445
   Gly Asn Glu Gln Ala Glu Asp Val Phe Tyr Ile Pro Ser Cys Met Ser
   450 455 460
   Gly Ser Arg Asn Phe Lys Met Ala Lys Leu Glu Asp Thr Arg Asn Val
   465 470 475 480
   Gly Met Pro Val Ala His Met Glu Ser Pro Glu Arg Tyr Leu His Leu
   485 490 495
   Lys Pro Thr Ile Glu Met Lys Asp Ser Val Pro Asn Lys Ala Gly Gly
   500 505 510
   Met Lys Asp Val Gln Thr Ser Lys Ala Ala Glu His Asp Leu Glu Val
   515 520 525
   Ala Ser Glu Glu Glu Gln Glu Arg Glu Gly Ser Glu Asn Asn Gln Pro
   530 535 540
   Gln Val Glu Glu Glu Arg Lys Lys His Arg Asn Asn Glu Met Glu Val
   545 550 555 560
   Ser Ala Asn Ile His Asp Gly Ala Thr Asp Asp Ala Glu Asp Asp Asp
   565 570 575
   Asp Asp Asp Gly Leu Ile Gln Lys Arg Lys Ser Gly Glu Thr Asp His
   580 585 590
   Gln Gln Phe Pro Arg Lys Glu Asn Lys Glu Tyr Ala Ser Ser Gly Pro
   595 600 605
   Ala Leu Gln Met Lys Glu Val Lys Ser Thr Glu Lys Glu Lys Arg Thr
   610 615 620
   Ser Lys Glu Ser Val Asn Ser Pro Val Phe Gly Lys Ala Ser Leu Leu
   625 630 635 640
   Thr Gly Gly Leu Leu Gln Val Asp Asp Asp Ser Ser Leu Ser Glu Ile
   645 650 655
   Asp Glu Asp Glu Gly Arg Pro Thr Lys Lys Thr Ser Asn Glu Lys Asn
   660 665 670
   Lys Val Lys Asn Gln Ile Gln Ser Met Asp Asp Val Asp Asp Leu Thr
   675                 680                 685
   Gln Ser Ser Glu Thr Ala Ser Glu Asp Cys Glu Leu Pro His Ser Ser
       690                 695                 700
   Tyr Lys Asn Phe Met Leu Leu Ile Glu Gln Leu Gly Met Glu Cys Lys
   705                 710                 715                 720
   Asp Ser Val Ser Leu Leu Lys Ile Gln Asp Ala Ala Leu Ser Cys Glu
                   725                 730                 735
   Arg Leu Leu Glu Leu Lys Lys Asn His Cys Glu Leu Leu Thr Val Lys
               740                 745                 750
   Ile Lys Lys Met Glu Asp Lys Val Asn Val Leu Gln Arg Glu Leu Ser
           755                 760                 765
   Glu Thr Lys Glu Ile Lys Ser Gln Leu Glu His Gln Lys Val Glu Trp
       770                 775                 780
   Glu Arg Glu Leu Cys Ser Leu Arg Phe Ser Leu Asn Gln Glu Glu Glu
   785                 790                 795                 800
   Lys Arg Arg Asn Ala Asp Thr Leu Tyr Glu Lys Ile Arg Glu Gln Leu
                   805                 810                 815
   Arg Arg Lys Glu Glu Gln Tyr Arg Lys Glu Val Glu Val Lys Gln Gln
               820                 825                 830
   Leu Glu Leu Ser Leu Gln Thr Leu Glu Met Glu Leu Arg Thr Val Lys
           835                 840                 845
   Ser Asn Leu Asn Gln Val Val Gln Glu Arg Asn Asp Ala Gln Arg Gln
       850                 855                 860
   Leu Ser Arg Glu Gln Asn Ala Arg Met Leu Gln Asp Gly Ile Leu Thr
   865                 870                 875                 880
   Asn His Leu Ser Lys Gln Lys Glu Ile Glu Met Ala Gln Lys Lys Met
                   885                 890                 895
   Asn Ser Glu Asn Ser His Ser His Glu Glu Glu Lys Asp Leu Ser His
               900                 905                 910
   Lys Asn Ser Met Leu Gln Glu Glu Ile Ala Met Leu Arg Leu Glu Ile
           915                 920                 925
   Asp Thr Ile Lys Asn Gln Asn Gln Glu Lys Glu Lys Lys Cys Phe Glu
       930                 935                 940
   Asp Leu Lys Ile Val Lys Glu Lys Asn Glu Asp Leu Gln Lys Thr Ile
   945 950 955 960
   Lys Gln Asn Glu Glu Thr Leu Thr Gln Thr Ile Ser Gln Tyr Asn Gly
   965 970 975
   Arg Leu Ser Val Leu Thr Ala Glu Asn Ala Met Leu Asn Ser Lys Leu
   980 985 990
   Glu Asn Glu Lys Gln Ser Lys Glu Arg Leu Glu Ala Glu Val Glu Ser
   995 1000 1005
   Tyr His Ser Arg Leu Ala Ala Ala Ile His Asp Arg Asp Gln Ser
   1010 1015 1020
   Glu Thr Ser Lys Arg Glu Leu Glu Leu Ala Phe Gln Arg Ala Arg
   1025 1030 1035
   Asp Glu Cys Ser Arg Leu Gln Asp Lys Met Asn Phe Asp Val Ser
   1040 1045 1050
   Asn Leu Lys Asp Asn Asn Glu Ile Leu Ser Gln Gln Leu Phe Lys
   1055 1060 1065
   Thr Glu Ser Lys Leu Asn Ser Leu Glu Ile Glu Phe His His Thr
   1070 1075 1080
   Arg Asp Ala Leu Arg Glu Lys Thr Leu Gly Leu Glu Arg Val Gln
   1085 1090 1095
   Lys Asp Leu Ser Gln Thr Gln Cys Gln Met Lys Glu Met Glu Gln
   1100 1105 1110
   Lys Tyr Gln Asn Glu Gln Val Lys Val Asn Lys Tyr Ile Gly Lys
   1115 1120 1125
   Gln Glu Ser Val Glu Glu Arg Leu Ser Gln Leu Gln Ser Glu Asn
   1130 1135 1140
   Met Leu Leu Arg Gln Gln Leu Asp Asp Ala His Asn Lys Ala Asp
   1145 1150 1155
   Asn Lys Glu Lys Thr Val Ile Asn Ile Gln Asp Gln Phe His Ala
   1160 1165 1170
   Ile Val Gln Lys Leu Gln Ala Glu Ser Glu Lys Gln Ser Leu Leu
   1175 1180 1185
   Leu Glu Glu Arg Asn Lys Glu Leu Ile Ser Glu Cys Asn His Leu
   1190 1195 1200
   Lys Glu Arg Gln Tyr Gln Tyr Glu Asn Glu Lys Ala Glu Arg Glu
   1205                 1210                 1215
   Val Val Val Arg Gln Leu Gln Gln Glu Leu Ala Asp Thr Leu Lys
       1220                1225                1230
   Lys Gln Ser Met Ser Glu Ala Ser Leu Glu Val Thr Ser Arg Tyr
       1235                1240                1245
   Arg Ile Asn Leu Glu Asp Glu Thr Gln Asp Leu Lys Lys Lys Leu
       1250                1255                1260
   Gly Gln Ile Arg Asn Gln Leu Gln Glu Ala Gln Asp Arg His Thr
       1265                1270                1275
   Glu Ala Val Arg Cys Ala Glu Lys Met Gln Asp His Lys Gln Lys
       1280                1285                1290
   Leu Glu Lys Asp Asn Ala Lys Leu Lys Val Thr Val Lys Lys Gln
       1295                1300                1305
   Met Asp Lys Ile Glu Glu Leu Gln Lys Asn Leu Leu Asn Ala Asn
       1310                1315                1320
   Leu Ser Glu Asp Glu Lys Glu Gln Leu Lys Lys Leu Met Glu Leu
       1325                1330                1335
   Lys Gln Ser Leu Glu Cys Asn Leu Asp Gln Glu Met Lys Lys Asn
       1340                1345                1350
   Val Glu Leu Glu Arg Glu Ile Thr Gly Phe Lys Asn Leu Leu Lys
       1355                1360                1365
   Met Thr Arg Lys Lys Leu Asn Glu Tyr Glu Asn Gly Glu Phe Ser
       1370                1375                1380
   Phe His Gly Asp Leu Lys Thr Ser Gln Phe Glu Met Asp Ile Gln
       1385                1390                1395
   Ile Asn Lys Leu Lys His Lys Ile Asp Asp Leu Thr Ala Glu Leu
       1400                1405                1410
   Glu Thr Ala Gly Ser Lys Cys Leu His Leu Asp Thr Lys Asn Gln
       1415                1420                1425
   Ile Leu Gln Glu Glu Leu Leu Ser Met Lys Thr Val Gln Lys Lys
       1430                1435                1440
   Cys Glu Lys Leu Gln Lys Asn Lys Lys Lys Leu Glu Gln Glu Val
       1445                1450                1455
   Ile Asn Leu Arg Ser His Ile Glu Arg Asn Met Val Glu Leu Gly
   1460                     1465                     1470
   Gln Val Lys Gln Tyr Lys Gln Glu Ile Glu Glu Arg Ala Arg Gln
       1475                     1480                     1485
   Glu Ile Ala Glu Lys Leu Lys Glu Val Asn Leu Phe Leu Gln Ala
       1490                     1495                     1500
   Gln Ala Ala Ser Gln Glu Asn Leu Glu Gln Phe Arg Glu Asn Asn
       1505                     1510                     1515
   Phe Ala Ser Met Lys Ser Gln Met Glu Leu Arg Ile Lys Asp Leu
       1520                     1525                     1530
   Glu Ser Glu Leu Ser Lys Ile Lys Thr Ser Gln Glu Asp Phe Asn
       1535                     1540                     1545
   Lys Thr Glu Leu Glu Lys Tyr Lys Gln Leu Tyr Leu Glu Glu Leu
       1550                     1555                     1560
   Lys Val Arg Lys Ser Leu Ser Ser Lys Leu Thr Lys Thr Asn Glu
       1565                     1570                     1575
   Arg Leu Ala Glu Val Asn Thr Lys Leu Leu Val Glu Lys Gln Gln
       1580                     1585                     1590
   Ser Arg Ser Leu Phe Thr Thr Leu Thr Thr Arg Pro Val Met Glu
       1595                     1600                     1605
   Pro Pro Cys Val Gly Asn Leu Asn Asn Ser Leu Asp Leu Asn Arg
       1610                     1615                     1620
   Lys Leu Ile Pro Arg Glu Asn Leu Val Ile Ser Thr Ser Asn Pro
       1625                     1630                     1635
   Arg Ala Ser Asn Asn Ser Met Glu Asn Tyr Leu Ser Lys Met Gln
       1640                     1645                     1650
   Gln Glu Leu Glu Lys Asn Ile Thr Arg Glu Leu Lys Glu Ala Ala
       1655                     1660                     1665
   Ala Glu Leu Glu Ser Gly Ser Ile Ala Ser Pro Leu Gly Ser Thr
       1670                     1675                     1680
   Asp Glu Ser Asn Leu Asn Gln Asp Leu Val Trp Lys Ala Ser Arg
       1685                     1690                     1695
   Glu Tyr Val Gln Val Leu Lys Lys Asn Tyr Met Ile
       1700                     1705                     1710

<210>  10

<211>  6816
<212>  DNA
<213>  homo sapiens

<400>  10
acttgtgtct agcgggatcg cttgcttggt aacccggagg gagagattgg aaaccgcgga      60

gtttcctttg ggaggctgcg gccagccggg gctgacttgt tatgttgggc tccggaggcc     120

gttaagagcc gagagagaca tgaggtgtct ctgaagcccg gtcgcctggg ccatgaagaa     180

gatttttagt aagaagggcg agtcgccctt gggctccttc gcgcggcggc agaggagcag     240

cgcgggaggc gggggcgagc cgggggaggg cgcctactcg cagcccggct accacgtccg     300

agaccgagat ctcggcaaga tccacaaagc tgccagcgcg ggtaatgtgg cgaaagtgca     360

gcagatcctt ttgctcagga agaatggctt gaacgataga gacaagatga acaggacggc     420

tctacatttg gcctgtgcca atggtcatcc agaagtagta actctcctgg tggacagaaa     480

atgccagctc aatgtctgtg acaacgaaaa caggacagct ctgatgaagg ctgtacaatg     540

ccaggaagag aaatgtgcaa ctattctgct agaacatggt gctgatccaa atcttgcgga     600

tgtccatggc aacactgctc ttcactatgc tgtctataat gaggacatat cagtagcaac     660

aaagctgctt ttgtatgatg caaatattga agcaaaaaac aaggatgacc tcacaccact     720

tttacttgca gtaagtggaa aaaagcagca aatggtggaa tttttaataa agaaaaaagc     780

aaatgtaaat gcagtagata agttggaaag cagtcaccaa ctaatttcag aatataaaga     840

agaaaggata cctaaacatt cttctcaaaa tagtaattca gtggatgaaa gctctgaaga     900

ctccttaagc aggctttctg gcaaaccggg tgttgatgat tcatggccta cctcagatga     960

cgaagacctc aattttgata ctaagaatgt cccaaaacca agcttagcaa agctaatgac    1020

tgcttctcag caatccagga aaaatttaga agcaacatat ggcactgtga gaacaggaaa    1080

tagaactttg tttgaggata gagattccga tagtcaagat gaagttgtgg ttgaaagcct    1140

tcctacaaca tcaatcaaag tccagtgctt ttctcatcct acctatcaat cacctgacct    1200

tcttccaaaa ccttcccaca agtcgttagc aaaccctggt cttatgaagg aagaaccaac    1260

aaagccaggc attgcaaaaa aagaaaatgg tattgatatt attgaaagtg ctccactaga    1320

gcaaacaaat aatgacaatt tgacttatgt tgatgaagtg cacaaaaata atagaagtga    1380

tatgatgtcc gcattaggat taggacaaga ggaagatata gaatcacctt gggattctga    1440

gagtatctct gagaattttc cacagaagta tgttgatcct ttagctgggg ctgcagacgg    1500

aaaagaaaaa aatataggaa atgaacaagc agaagatgtg ttttatatac cttcttgcat    1560

gagtggatca agaaacttta agatggctaa actagaggat acaagaaatg taggcatgcc    1620

agtagcccac atggagtctc ctgagagata tcttcacttg aagcctacca ttgaaatgaa    1680

agattctgtt ccaaataaag caggaggaat gaaggatgta caaacatcca aagcagctga    1740

acatgactta gaagtagcat cagaagaaga gcaagaaagg gaagggagtg aaaataacca    1800

gccacaggtt gaagaagaaa ggaaaaaaca cagaaataat gaaatggaag tatcagcaaa    1860

catacatgat ggtgctactg atgatgctga agatgatgat gatgatgatg gattaattca    1920

aaaaagaaag agtggagaaa ctgatcatca gcaatttccc aggaaggaaa ataaagagta 1980
tgctagtagt ggtcctgcct tgcaaatgaa ggaagtaaag agcactgaaa aagaaaaacg 2040
gacctcgaaa gaatctgtga attcaccagt gtttgggaag gccagtttac taactggtgg 2100
cctgctacaa gtggatgatg acagcagttt aagtgaaata gatgaggatg aaggaaggcc 2160
tactaagaaa acatctaatg aaaagaacaa ggtcaaaaac caaatacagt ctatggatga 2220
tgttgatgac ttaactcagt catctgaaac agcctcagag gattgtgagc taccccactc 2280
tagttacaag aattttatgt tgctcattga acaacttgga atggagtgta aagattctgt 2340
tagcctattg aaaatccagg atgcagctct ttcatgtgaa agattattag aacttaaaaa 2400
aaatcactgt gaactactta cagtaaaaat taaaaaaatg gaagacaagg ttaatgtact 2460
acaaagggag ctatctgaaa caaaagaaat aaaatcacag ttagagcatc aaaaagttga 2520
atgggaacga gaactgtgct ctttgagatt tagcttaaac caagaagaag agaagagaag 2580
aaatgctgat acgttgtatg aaaaaattag ggaacagtta agaagaaaag aagagcaata 2640
taggaaagaa gttgaagtga aacaacagct tgaactgagt ctccaaacac tggagatgga 2700
attgaggact gtaaaaagta atttgaatca ggtcgttcaa gagcgaaatg acgctcagag 2760
gcaactttct cgagaacaga atgccagaat gttacaagat ggaattctga ccaatcacct 2820
ttccaaacaa aaggagattg aaatggctca aaagaaaatg aattctgaga attctcatag 2880
tcatgaagaa gaaaaagacc tatcgcataa aaatagcatg ttgcaggaag aaattgctat 2940
gctaagacta gaaatagaca caataaaaaa tcaaaaccag gaaaaagaaa agaaatgttt 3000
tgaggacctt aaaattgtaa aagaaaagaa tgaagacctt cagaagacta taaaacagaa 3060
tgaggaaaca ttaacacaaa caatatccca gtataatgga cggcttagtg ttctgacagc 3120
tgagaatgca atgctaaatt ctaaactgga gaatgaaaag caaagcaagg aaagactgga 3180
agcagaagtt gaatcatacc attctagatt ggctgctgct atacatgatc gtgatcaaag 3240
tgagacatca aaaagagaac tagaacttgc tttccagaga gcaagagatg aatgttctcg 3300
tttacaggac aaaatgaatt ttgatgtgtc taacctaaaa gataacaatg agattctttc 3360
tcaacaacta tttaaaactg aaagtaaact caatagccta gaaattgagt tccatcacac 3420
gagagatgcc ctcagagaaa agactttggg tttagaacgg gtacaaaagg acctaagcca 3480
aacacagtgt caaatgaagg aaatggaaca aaagtatcaa aatgaacaag ttaaagtgaa 3540
taaatacatt ggaaagcagg agtctgtaga ggagagattg tctcaactac aaagtgagaa 3600
tatgttgctt cgacaacaac tggatgatgc ccacaacaag gctgacaata aagagaagac 3660
agtgattaat atccaagacc agtttcatgc tattgtgcaa aaacttcaag ctgagagtga 3720
aaagcaaagt cttctgctag aagaaagaaa taaggagtta atcagtgaat gtaatcactt 3780
aaaagaaaga cagtatcaat atgaaaatga aaaggcagaa agagaagttg ttgtgagaca 3840
acttcaacaa gaactagctg ataccctaaa aaaacaatct atgtcagagg cttcactgga 3900
ggttacgtca cgttatcgta ttaatttaga agatgagaca caggatttaa agaagaaatt 3960

aggtcaaatc agaaatcaat tgcaagaagc acaggatcga catacagaag ctgtcagatg 4020
tgctgagaag atgcaagatc acaagcaaaa gcttgaaaaa gataatgcca agttaaaagt 4080
tacagtcaaa aagcaaatgg acaaaattga ggagcttcag aaaaacctgt taaatgcaaa 4140
tttgtctgaa gatgaaaagg aacaattaaa gaaacttatg gaattaaaac agtcactgga 4200
atgtaatttg gatcaagaaa tgaagaaaaa tgttgaatta gaaagagaga taactggatt 4260
taagaacctc ttaaaaatga caagaaagaa gttaaatgaa tatgaaaatg gagaatttag 4320
tttccatgga gatttaaaaa ctagtcaatt tgaaatggat attcagatta ataagctaaa 4380
acataagatt gatgatctta cagcagaact ggagactgca ggttcaaaat gtctacatct 4440
ggatacaaag aaccaaattc ttcaagagga gttgttatct atgaaaacag tacaaaagaa 4500
atgtgaaaaa ctacagaaga ataaaaagaa gttggaacaa gaagtgatca acctgagaag 4560
tcatatagaa aggaatatgg tagaacttgg tcaagtcaaa cagtataaac aggagattga 4620
agaaagagca agacaggaaa tagcagaaaa attaaaagaa gtcaatctat ttttacaggc 4680
acaagcagca tctcaagaaa acttagagca gtttagagag aataattttg cttcaatgaa 4740
aagtcagatg gaactcagaa ttaaagatct ggaatctgaa ctctccaaaa taaaaacttc 4800
tcaagaagac tttaataaaa ccgaactgga aaaatataag caactctatc tagaagaatt 4860
aaaagttaga aaatctttgt caagtaaact aaccaaaact aatgagaggc tagcagaggt 4920
caacaccaaa cttcttgtgg aaaaacagca gagcagatct ttgttcacca ctctcactac 4980
caggccagtc atggagccac cttgtgtggg aaatcttaat aatagtttag atctcaacag 5040
aaaacttatt ccaagagaaa acttagtgat ctctacctca aatccacggg cttcaaataa 5100
tagcatggag aactacttga gcaagatgca gcaggagttg gaaaaaaata taactagaga 5160
actcaaagaa gctgctgctg aattggaatc tggatcaata gcttcccctc tagggtctac 5220
tgatgagtca aatctaaatc aagatctagt ttggaaagca tcaagagaat atgtacaggt 5280
tttaaagaaa aattatatga tctgaaagat aagattttat tactgggctg tttatgtgac 5340
attttgtttc tcattaaata ttaatatatg acatgtaaaa atattaaagg aacgtatttt 5400
tgtatcatat atgtcagatc aaaatttata tagtatttta aacaatgtta cttggcttct 5460
ttaactttta agtggatttt gccaatgaaa accaggaata ttgagtttta catactcaaa 5520
ctgcccaaat gtgagctgtt taaacagcca aaccactaag ttgtcattaa tactttagtg 5580
cagttaattg atggcttatt tgtattttgt aatttttatc actatatgta gtggtagatg 5640
tctaattgga cttagacatc attttgttgt gcaggaactg tggtcattgt tcatgtttgg 5700
atgtattaca attgttacag tgccatcaaa tttacataat tttaatttta agcactgatt 5760
tatatagctg ttctcaggga gaaacaagat ttgcctgatt caccttttct gtttgtttat 5820
ttatttttaa ttaacctttt ggaaatagcc ttcagtttag agaaaatttc gaagttttag 5880
tacaaagact ttctcctccg aacaatttga gagtaaattg ctgacattat gtcatcaccc 5940
ctgattactt tagtgggcat tcctacaagt caggacattc tacataacta cagtgcaaac 6000

cttgcaatca gaaagtcaac attaatacat tctagtttgt tacttctctg taatgtcctt 6060
tatagcaaaa ggatcccatt ctaatccatg gcttgtattt agttgttgta tctgttagat 6120
ctttcagtct ggaacagttc ctcagtcttt ccttgacctt catgaccttg aatattttga 6180
agagtaaagt ctggtaatat aagaatgtcc ctcaattggg tttcatggtg attagattta 6240
gattatatga ttttttggca ggagtatcac agaagtgacc cagtgatctt ctcattgcat 6300
cttacctgtg accctacagt tttgatttgc cctgttactg gtaatgttag cattgatcac 6360
taacagtggt atctgctgaa tttctcctct gtaatgttat tttttcacct tgtggttaat 6420
tagtatttta cagggaggta atttgagact atgtacatat ctctactccc catcgaaccc 6480
tcaatttatt catttgcttg tatcaaataa accatggatt catggtttat tttattcagt 6540
gagttataat tcattaatat tgttattaaa aatttgtttt tcttttaaga aatgttatat 6600
tttgacaact tcagacttac agaaagatta taagaggtag tacaaataat ttttggatat 6660
tattccccaa atgttaacat tttactgcat ttactttatc ctttctcccc cttctcctcc 6720
tgtctttcta gatgaatatg aatataggta cttaatacag attttttttc taaactgtta 6780
aaaaaaaaaa aaaaaaaaaa aaaaaaaaaa aaaaaa 6816

## Description

### FIELD OF THE INVENTION

The present invention relates to ANKRD26 as a biomarker for diagnosing thrombocytopenias such as THC2 and FPD/AML.

### BACKGROUND OF THE INVENTION

Inherited thrombocytopenias constitute a heterogeneous group characterized by a reduced number of blood platelets and a bleeding tendency ranging from very mild to life threatening. The genetic defects associated with the pathology have been identified for some of these pathologies, whereas they are still unknown for the others. These defects generally consist in a mutation in a gene implied in the process of megakaryopoiesis and platelet production.

A significant proportion of patient affected by an inherited thrombocytopenia remain without a definite diagnosis. Making a definitive diagnosis for these patients remains important because the different forms of these pathologies imply different prognosis, therapy and management. Indeed, for example, some inherited thrombocytopenias, such as familial platelet disorder with propensity for myeloid malignancy (FPD/AML) or thrombocytopenia 2 (THC2), significantly increase the risk of leukemia.

Thrombocytopenia 2 (THC2; OMIM 188000) is one of the rarest form of familial autosomal-dominant thrombocytopenias, for which mutations of MASTL and ACBD5 genes have been identified as causative defects. THC2 is characterized by a moderate thrombocytopenia, reduction of platetlet α-granules, normal platelet aggregation and normal mean platelet volume, and seems to be derived from dysmegakaryopoiesis. Recently, mutations in a short stretch of the 5'-untranslated region (5'-UTR) of the ANKRD26 gene have been identified and associated with this particular form of thrombocytopenia (Pippucci T. et al, The American Journal of Human Genetics 88, 115-120, January 7, 2011; Norris P; et al, Blood, 16 June 2011, Volume 117, Number 24): these studies indicate that the ANKRD26 mutations cause thrombocytopenia and might enhance ANKRD26 expression.

Familial platelet disorder with propensity to acute myeloid leukaemia (FPD/AML; OMIM 601399) is also an autosomal-dominant disorder in which affected members have a clinical history of bleeding tendency and mild to moderate thrombocytopenia with normal platelet size and morphology, and/or abnormal platelet aggregation in response to arachidonic acid (HO et al., Blood, vol.87, p: 5218-5224, 1996). FPD/AML has been associated with RUNX1 (Song et al., Nat Genet, vol.23, p: 166-175, 1999; Michaud et al., Blood, vol.99, p :1364-1372, 2002). FPD/AML diagnosis is currently based on sequencing the complete RUNX1 gene sequence and new biomarkers of the pathology recently appeared such as MYH10 (Antony-Debré I et al, Blood. 2012 Jun 7).

FPD/AML and THC2 are both familial autosomal-dominant thrombocytopenias characterized by the same clinical symptoms and a predisposition to leukemias. It would be of great interest to dispose of methods or biomarkers permitting a simple diagnosis, and more particularly a differential diagnosis, of these both pathologies.

### SUMMARY OF THE INVENTION

The present inventors interestingly discovered that the ANKRD26 protein is present in platelets of THC2 and FPD/AML patients, whereas it is absent in platelets of healthy subjects. They also showed that MYH10 is present in platelets of FPD/AML patients, whereas it is absent in platelets of THC2 patients and healthy subjects.

Thus, the present invention relates to an *in vitro* method for diagnosing thrombocytopenia in a subject, said method comprising the step of (i) assessing the ANKRD26 platelet expression in a biological sample from said subject, and wherein an ANKRD26 platelet expression is indicative of a thrombocytopenia.

Particularly, the invention relates to an *in vitro* method as described above, wherein said method is used for distinguishing THC2 and FPD/AML and said method further comprises step of (ii) assessing the MYH10 platelet expression in a biological sample from said subject, and wherein:
a. an ANKRD26 platelet expression and no MYH10 platelet expression is indicative of a THC2, and
b. an ANKRD26 platelet expression and a MYH10 platelet expression is indicative of a FPD/AML.

The invention also provides a kit for diagnosing a thrombocytopenia in a subject according to the methods of the invention, which comprises (i) at least one ANKRD26 nucleic acid probe or oligonucleotide or antibody for determining the ANKRD26 platelet expression in a biological sample from said subject.

Particularly, the invention provides a kit as described above, wherein said kit further comprises (ii) at least one MYH10 nucleic acid probe or oligonucleotide or antibody for further assessing the MYH10 platelet expression in a biological sample from said subject.

### BRIEF DESCRIPTION OF DRAWINGS

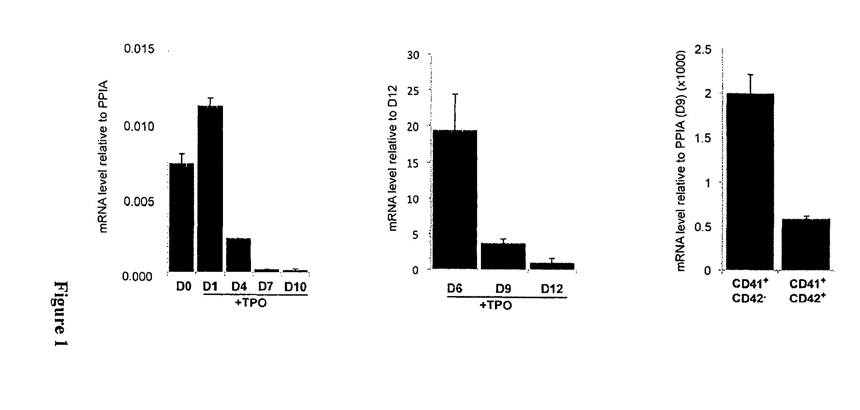

The **Figure 1** shows **ANKRD26 expression level during megakaryocyte differentiation.** CD34+ cells (D0) were obtained from cytapheresis and cultures in presence of thrombopoietin (TPO) alone to induce megakaryocyte differentiation. The cells were collected at day 1, 4, 7 and 10 of culture in the experiment represented in the left panel, at day 6, 9 and 12 of culture in the experiment represented in the middle panel and megakaryocytes were sorted according to CD41 and CD42 expression at day 6 of culture in the experiment represented in the right panel. CD41+CD42- represent the immature megakaryocyte CD41+CD42+ represent more mature megakaryocytes. The RNA expression level of ANKRD26 was measured by Q-RT-PCR and its level is represented relative to PPIA.

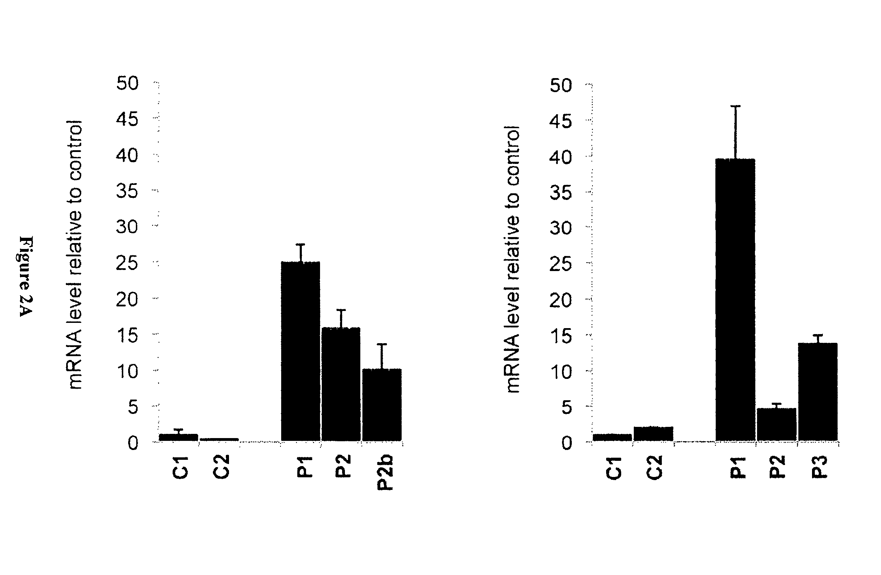

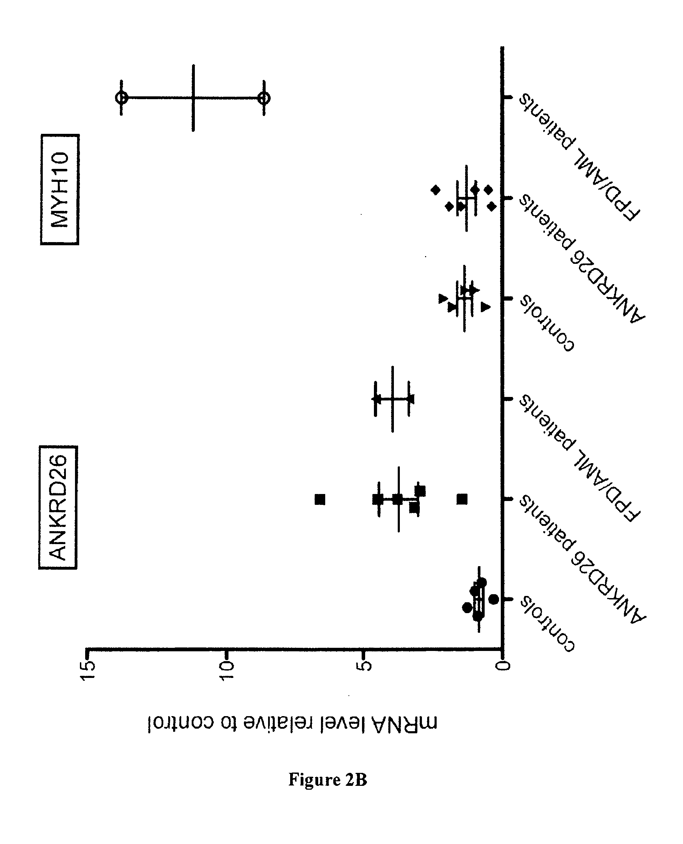

The **Figure 2** shows **ANKRD26 expression in megakaryocytes of THC2 patients and platelets of THC2 and FPD/AML patients.**

**Figure 2A****:** CD34⁺ cells were isolated fromTHC2 patient's (P1, P2, P3) or healthy individual's (C1, C2) peripheral blood and cultured in presence of thrombopoietin. CD41⁺CD42⁺ megakaryocytes were sorted at day 10 of culture and ANKRD26 expression level was measured by Q-RT-PCR, normalized to PPIA and expressed relatively to C1. P2 and P2b represent two different RNA extractions.

**Figure 2B****:** THC2 patient's (ANKRD26 patients, P1-P6), FPD/AML patient's (RUNX1 mutations, P1, P2) and healthy individual's (C1-C5) platelets were obtained from platelets-rich plasma. ANKRD26 and MYH10 expression level was measured by Q-RT-PCR, normalized to PPIA and expressed relatively to C1.

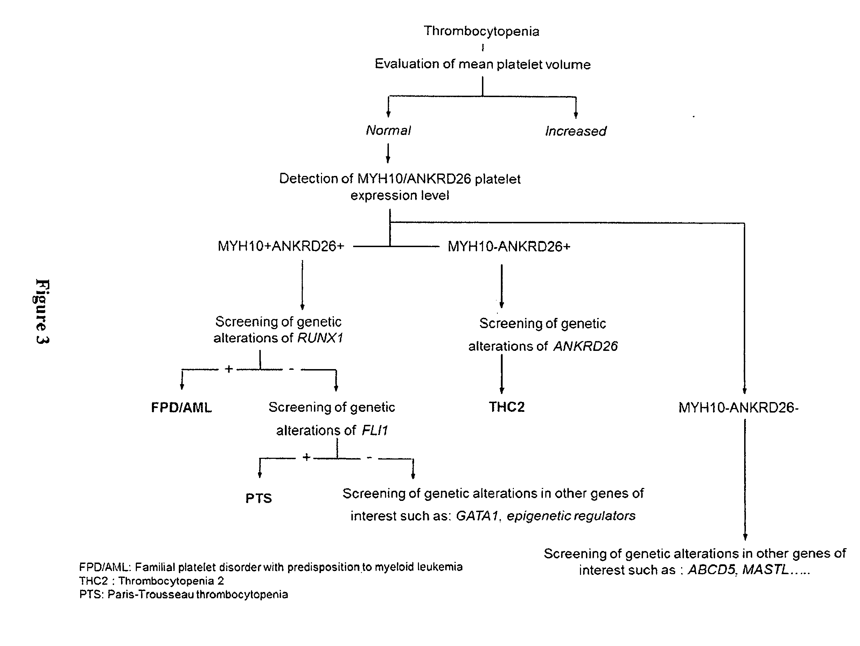

The **Figure 3** illustrates a **diagram of the double screening of MYH10 and ANKRD26 expression to distinct between the pathologies presenting similar clinical phenotype.**

### DETAILED DESCRIPTION OF THE INVENTION

**Methods of the invention**

A first object of the invention relates to an *in vitro* method for diagnosing thrombocytopenia in a subject, said method comprising the step of (i) assessing the ANKRD26 platelet expression in a biological sample from said subject, and wherein an ANKRD26 platelet expression is indicative of a thrombocytopenia.

The term "ANKRD26" has its general meaning in the art and refers to a gene encoding in humans the *Ankyrin repeat domain-containing protein* 26 or ANKRD26 protein (also called THC2 gene or bA145E8.1). The term may include naturally occurring ANKRD26 and variants and modified forms thereof. The ANKRD26 can be from any source, but typically is a mammalian (e.g., human and non- human primate) ANKRD26, preferably a human ANKRD26. An exemplary native ANKRD26 amino acid sequence is provided in GenPept database under accession number NP_001242982 (SEQ ID NO :1) or NP_055730 (SEQ ID NO:9) and an exemplary native nucleotide sequence encoding for ANKRD26 is provided in GenBank database under accession number NM_001256053 (SEQ ID NO :2) or NM_014915 (SEQ ID NO:10).

Thrombocytopenias are well known from the skilled person and include *familial thrombocytopenia 2* called THC2 and familial platelet disorder with propensity to acute myeloid leukemia called FPD/AML. These both forms of thrombocytopenia are associated with a predisposition to leukemia such as acute myeloid leukemia (AML) and chronic myelomonocytic leukemia (CMML).

In a particular embodiment, thrombocytopenia is THC2. In another embodiment, thrombocytopenia is FPD/AML.

As used herein, the term "subject" denotes a mammal, such as a rodent, a feline, a canine, and a primate. Preferably, a subject according to the invention is a human.

As used herein, the term "biological sample" refers to a sample in which platelets ANKRD26 expression level may be assayed. Biological samples comprising platelets are well known from the skilled person and include blood samples and bone marrow samples. Preferably, the biological sample is a blood sample.

In a particular embodiment, the *in vitro* method of the invention is used for distinguishing THC2 and FPD/AML. It further comprises a step of (ii) assessing the MYH10 platelet expression in said biological sample (or in a biological sample obtained from said subject).

In this particular embodiment, the combination of (a) a ANKRD26 platelet expression and (b) no MYH10 platelet expression is indicative of THC2; the combination of (a') a ANKRD26 platelet expression and (b') a MYH10 platelet expression is indicative of FPD/AML.

The term "MYH10" has its general meaning in the art and refers to the gene encoding the myosin non-muscle heavy chain 10 protein or MYH10 protein (Gene ID: 4628, also known as NMMHCB, MGC134913 or MGC134914). The term may include naturally occurring MYH10 and variants and modified forms thereof. The MYH10 can be from any source, but typically is a mammalian (e.g., human and non- human primate) MYH10, preferably a human MYH10. An exemplary native MYH10 amino acid sequence is provided in GenPept database under accession number NP_005955 (SEQ ID NO: 3) and an exemplary native nucleotide sequence encoding for MYH10 is provided in GenBank database under accession number NM_005964 (SEQ ID NO: 4).

The platelets expression level of a gene or a protein in a biological sample of the invention may be assessed by purifying platelets from said biological sample.

Methods for purifying platelets from biological samples such as blood samples are well known from the skilled person and are disclosed, as an example, in NIUL et al. (Comparative Haematology International, vol.2(2), p: 106-110, 1992), GNATENKO et al. (Methods Mol. Biol., vo1.496, p:245-72, 2009), in SCHEDEL & ROLF (Methods Mol. Biol., vo1.496, p:273-83, 2009), or in CHOI et al. (Lab. Chip., "Hydrophoretic high-throughput selection of platelets in physiological shear-stress range", 2010).

The subject may be healthy, but the method of the invention is particularly useful for testing a subject thought to develop or to be predisposed to developing thrombocytopenia chosen among the group comprising THC2 and FPD/AML. In that case, the method of the invention enables to confirm that said subject develops or is predisposed for developing THC2 or FPD/AML.

In a particular embodiment, the method of the invention may be used for prognosing a predisposition to AML or CMML in a subject.

In a preferred embodiment, the method of the invention further comprises the step of comparing said ANKRD26 and/or MYH10 platelet expression in said biological sample with a control.

As used herein said "control" refers to the platelet ANKRD26 and/or MYH10 expression level in a control sample corresponding to the ANKRD26 and/or MYH10 expression level in platelets of a biological sample from a healthy subject. Said control is preferably the average expression levels of ANKRD26 and/or MYH10 in several control biological samples. As determined by the inventors, there is no expression of ANKRD26 and/or MYH10 in the platelets of healthy subjects.

Thus, as used herein, an "ANKRD26 and/or MYH10 platelet expression" means a significant ANKRD26 and/or MYH10 platelet expression (or expression level) compared to said control expression (or expression level), by any method for assessing a platelet ANKRD26 and/or MYH10 expression (or expression level).

A significant ANKRD26 and/or MYH10 platelet expression (or expression level) compared to said control level refers to an expression at least two, particularly at least three, more particularly at least five, preferably at least ten times higher than the ANKRD26 and/or MYH10 platelet control expression (or expression level).

Methods for determining the ANKRD26 and/or MYH10 expression levels in platelets are well known for the man skilled in the art.

In one particular embodiment, the level of expression of the ANKRD26 and/or MYH10 gene(s) is assessed by determining the level of expression of the ANKRD26 protein (i.e. SEQ ID N°1 or SEQ ID N°9) and/or the MYH10 protein (i.e. SEQ ID N°3).

Methods for determining the level of expression of a protein are well known in the art. Such methods comprise contacting a biological sample with a binding partner capable of selectively interacting with the protein of interest present in the sample. The binding partner is generally an antibody.

Thus, such analysis can be assessed an antibody (e.g., a radio-labeled, chromophore-labeled, fluorophore-labeled, or enzyme-labeled antibody), an antibody derivative (e.g., an antibody conjugate with a substrate or with the protein or ligand of a protein of a protein/ligand pair (e.g., biotin-streptavidin)), or an antibody fragment (e.g., a single-chain antibody, an isolated antibody hypervariable domain, etc.) which binds specifically to the ANKRD26 and/or MYH10 protein respectively. Said analysis can be assessed by a variety of techniques well known by one of skill in the art including, but not limited to, enzyme immunoassay (EIA), radioimmunoassay (RIA), Western blot analysis and enzyme linked immunoabsorbant assay (ELISA).

Polyclonal antibodies can be prepared by immunizing a suitable animal, such as mouse, rabbit or goat, with the ANKRD26 protein (SEQ ID NO: 1 or SEQ ID N°9) and/or MYH10 protein (SEQ ID NO:3) or a fragment thereof (e.g., at least 10 or 15 amino acids). The antibody titer in the immunized animal can be monitored over time by standard techniques, such as with an ELISA using immobilized polypeptide.

ANKRD26 or MYH10 antibodies are already commercially available from SANTA CRUZ BIOTECHNOLOGY, INC., LIFESPAN BIOSCIENCES, or from SIGMA-ALDRICH.

At an appropriate time after immunization, e.g., when the specific antibody titers are highest, antibody producing cells can be obtained from the animal and used to prepare monoclonal antibodies (mAb) by standard techniques, such as the hybridoma technique originally described by KOHLER and MILSTEIN (Nature, vol.256, p:495-497, 1975), the human B cell hybridoma technique (KOZBOR et al., Immunol., vol.4, p: 72, 1983), the EBV- hybridoma technique (COLE et al., In Monoclonal Antibodies and Cancer Therapy, Alan R. Liss,Inc., p: 77-96, 1985) or trioma techniques. The technology for producing hybridomas is well known (see generally Current Protocols in Immunology, COLIGAN et al. ed., John Wiley & Sons, New York, 1994). Hybridoma cells producing the desired monoclonal antibody are detected by screening the hybridoma culture supernatants for antibodies that bind the polypeptide of interest, e.g., using a standard ELISA.

In another particular embodiment of the invention, the level of expression of the *ANKRD26* and/or *MYH10* gene is assessed by determining the level of expression of its mRNA transcript (i.e., SEQ ID N°2 or SEQ ID N°10 for *ANKRD26* and SEQ ID NO:4 for *MYH10*) or mRNA precursors, such as nascent RNA, of said gene.

Methods for determining the quantity of mRNA are well known in the art.

Such analysis can be assessed by preparing mRNA/cDNA from cells in a biological sample from a subject, and hybridizing the mRNA/cDNA with a reference polynucleotide. The prepared mRNA/cDNA can be used in hybridization or amplification assays that include, but are not limited to, Southern or Northern analyses, polymerase chain reaction analyses, such as quantitative PCR (TAQMAN), and probes arrays such as GENECHIP^{™} DNA Arrays (AFFYMETRIX).

Advantageously, the analysis of the expression level of mRNA transcribed from the *ANKRD26* and/or *MYH10* gene involves the process of nucleic acid amplification, e. g., by RT-PCR (the experimental embodiment set forth in U. S. Patent No. 4,683, 202), ligase chain reaction (BARANY, Proc. Natl. Acad. Sci. USA, vol.88, p: 189-193, 1991), self sustained sequence replication (GUATELLI et al., Proc. Natl. Acad. Sci. USA, vol.87, p: 1874-1878, 1990), transcriptional amplification system (KWOH et al., 1989, Proc. Natl. Acad. Sci. USA, vol.86, p: 1173-1177, 1989), Q-Beta Replicase (LIZARDI et al., Biol. Technology, vol.6, p: 1197, 1988), rolling circle replication (U. S. Patent No. 5,854, 033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3'regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

Preferably, a pair of primers permitting to amplify ANKRD26 has the sequences SEQ ID N°5 and SEQ ID N°6 and a pair of primers permitting to amplify MYH10 has the sequences SEQ ID N°7 and SEQ ID N°8.

In a preferred embodiment, the invention relates to an *in vitro* method for diagnosing THC2 in a subject, said method comprising the step of (i) assessing the ANKRD26 platelet expression in a biological sample from said subject, and wherein a ANKRD26 platelet expression is indicative of THC2.

In a more preferred embodiment, said method further comprises a step of (ii) assessing the MYH10 platelet expression in said biological sample (or in a biological sample obtained from said subject), wherein no MYH10 platelet expression is indicative of THC2.

**Kits of the invention**

A second object of the invention relates to a kit for diagnosing a thrombocytopenia in a subject according to the methods of the invention, which comprises (i) at least one mean for assessing the ANKRD26 platelet expression in a biological sample from said subject.

In a particular embodiment, said kit comprises an ANKRD26 nucleic acid probe or oligonucleotide or antibody for assessing the ANKRD26 platelet expression in a biological sample from said subject.

Preferably, the oligonucleotide is at least one PCR primer pair, most preferably SEQ ID NO: 5 and SEQ ID NO: 6.

In one embodiment, the kit of the invention further comprises (ii) at least one mean for assessing the MYH 10 platelet expression in a biological sample from said subject.

In a particular embodiment, said kit comprises a MYH10 nucleic acid probe or oligonucleotide or antibody for further assessing the MYH10 platelet expression in a biological sample from said subject.

Preferably, the oligonucleotide is at least one PCR primer pair, most preferably SEQ ID NO: 7 and SEQ ID NO: 8.

The kit of the invention can further comprise at least one mean for purifying platelets from a biological sample, preferably from a blood sample.

The kit of the invention may be used for diagnosing a thrombocytopenia in a subject according to the methods of the invention, wherein said thrombocytopenia is preferably THC2 or FPD/AML. The invention thus relates to the use of a kit of the invention for diagnosing a thrombocytopenia, preferably THC2 or FPD/AML.

In a particular embodiment, the kit of the invention may be used for testing a subject thought to develop or to be predisposed to developing a thrombocytopenia, wherein said thrombocytopenia is preferably FPD/AML or THC2. The invention thus relates to the use of a kit of the invention for testing a subject thought to develop or to be predisposed to developing a thrombocytopenia, preferably THC2 or FPD/AML.

In another particular embodiment, the kit of the invention may be used for prognosing a predisposition to AML and/or CMML in a subject. The invention thus relates to the use of a kit of the invention for prognosing a predisposition to AML and/or CMML in a subject.

As used herein, the term "kit" refers to any delivery system for delivering materials. In the context of reaction assays, such delivery systems include systems that allow for the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to delivery systems comprising two or more separate containers that each contains a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

The present kits can also include one or more reagents, buffers, hybridization media, nucleic acids, primers, nucleotides, probes, molecular weight markers, enzymes, solid supports, databases, computer programs for calculating dispensation orders and/or disposable lab equipment, such as multi-well plates, in order to readily facilitate implementation of the present methods. Enzymes that can be included in the present kits include nucleotide polymerases and the like. Solid supports can include beads and the like whereas molecular weight markers can include conjugable markers, for example biotin and streptavidin or the like.

In one embodiment, the kit is made up of instructions for carrying out the method described herein. The instructions can be provided in any intelligible form through a tangible medium, such as printed on paper, computer readable media, or the like.

In the following, the invention is described in more detail with reference to amino acid sequences, nucleic acid sequences and the examples. Yet, no limitation of the invention is intended by the details of the examples. Rather, the invention pertains to any embodiment which comprises details which are not explicitly mentioned in the examples herein, but which the skilled person finds without undue effort.

### EXAMPLES

### MATERIAL AND METHODS

**Patients.**

The study was approved by the Local Research Ethics Committee from AP-HP (Assistance Publique des Hôpitaux de Paris) and INSERM. Blood samples from patients and healthy subjects were collected with informed consent in accordance with the Declaration of Helsinki.

**In vitro megakaryocyte culture derived from human CD34+ cells in liquid serum-free medium.**

CD34+ cells and platelets were obtained in agreement with our Institute Ethic Committee (AP-HP) from the peripheral blood of healthy patients undergoing hip surgery and from leukapheresis samples after mobilization. CD34+ cells were isolated by an immunomagnetic cell sorting technique (AUTOMACS; MILTENYI BIOTEC, Bergisch Gladbach, Germany) and cultured in serum-free medium in the presence of recombinant human thrombopoietin (TPO, 10 ng/mL; KIRIN BREWERY, Tokyo, Japan) for inducing megakaryocyte differentiation. Megakaryocytes were sorted according to CD41 and CD42 expression using a FACSDIVA or Influx flow cytometer (BECTON DICKINSON, Mountain View, CA).

**Platelets**

The platelet-rich plasma was prepared by centrifugation at 900 rpm for 10 min. Platelets were pelleted by centrifugation at 3,000 rpm for 10 min.

**Quantitative real-time PCR (RT-PCR).**

RNA was isolated using micro RNeasy extraction kit (QUIAGEN), reverse-transcribed by Vilo Superscript kit (INVITROGEN) and Q-RT-PCR were carried out with a PRISM® 7700 sequence detection system using SYBR® green (APPLIED BIOSYSTEMS). The expression levels of all genes studied were expressed relatively to housekeeping genes PPIA.

**Western Blot.**
The proteins are extracted from megakaryocytes at day 6, 9 and 12 of culture in presence of TPO, from platelets of healthy controls and THC2 and FPD/AML patients. The western blot is performed as previously described (Antony-Debré et al, Blood, 2012) using antibodies directed against ANKRD26 (SANTA CRUZ BIOTECHNOLOGY), MYH10 (CELL SIGNALING), and HSC70 (SIGMA). The protein expression level of MYH10 is normalized to HSC70. Super Signal West Femto Maximum Sensitivity Substrate (Thermo Scientific) is used for MYH10 detection and Immobilon Western Chemiluminescent HRP Substrate (Millipore Corporation) for HSC70 detection.

**Statistics.**

Data are presented as means (±SD). Statistical significance was determined by Student's t test. A P value < 0.05 was considered as statistically significant.

### RESULTS

**ANKRD26 expression during normal megakaryocyte differentiation.**

Inventors demonstrated that in hematopoietic progenitors (CD34+ cells corresponding to D0), a high level of ANRD26 was detected.

After induction of megakaryopoiesis by thrombopoietin **(****Figure 1****),** the decrease in ANKRD26 expression was detected by Q-RT-PCR. At the end of megakaryocyte culture (D10-D12), the ANKRD26 was quasi absent.

The ANKRD26 was also measured within two different megakaryocyte populations (CD41+CD42- corresponding to immature megakaryocyte and CD41+CD42+ corresponding to more mature megakaryocyte) at the same day of culture (D6). As shown in **Figure 1** (right panel), the level of ANKRD26 was decreased in more mature megakaryocyte compared to the immature megakaryocyte.

These three independent experiments represented on the **Figure 1** (left, middle and right panel) confirmed the downregulation of ANKRD26 during normal megakaryocyte differentiation.

**ANKRD26 persist in megakaryocytes and platelets of THC2 patients.**

Inventors further tested the hypothesis that ANKRD26 should persist in patient megakaryocytes and platelets of THC2 patients.

CD34+ hematopoietic progenitors obtained from peripheral blood of three patients (P1 with c.-119C>A mutation, P2 and P3 with c-127A>T mutation) were grown in the presence of thrombopoietin to induce the megakaryocyte differentiation. At day 10 of culture, the mature megakaryocytes (CD41+CD42+ cells) were sorted and the level of ANKRD26 was analysed by Q-RT-PCR. As shown in **figure 2A****,** the increased ANKRD26 expression was detected in the megakaryocytes of all three patients (P1, P2 and P2b - two different extractions for P2 at left and P1, P2 and P3 at right) compared to control megakaryocyte.

Then, platelets were obtained from platelet-rich plasma of 6 patients from 3 pedigrees (with c.-119C>A, c-127A>T, c.-118C>A mutations) and ANKRD26 level was detected by Q-RT-PCR. As shown in **Figure 2B****,** a significantly increased level of ANKRD26 was detected in platelets of all patients.

**ANKRD26 also persist in megakaryocytes and platelet of FPD/AML patients.**

The inventors further similarly showed that the ANKRD26 level was also increased in platelets of FPD/AML patients **(****Figure 2B****).**

Previously inventors reported that MYH10 is deregulated in FPD/AML megakaryocytes and platelets in the same manner as ANKRD26 in THC2.

In order to precise the expression pattern of these both biomarkers in thrombocytopenias, inventors analysed the MYH10 expression in platelets of THC2 patients and showed that MYH10 was absent from THC2 platelets **(****Figure 2B****).**

**Assessment of the MYH10 and/or ANKRD26 protein level.**

As described before, CD34+ hematopoietic progenitors obtained from peripheral blood of three patients (P1 with c.-119C>A mutation, P2 and P3 with c-127A>T) are grown in the presence of thrombopoietin to induce the megakaryocyte differentiation. At day 10 of culture, the mature megakaryocytes (CD41+CD42+ cells) are sorted and the level of ANKRD26 and/or MYH10 protein(s) is analysed by western blot or ELISA.

An increased ANKRD26 expression is detected in the megakaryocytes of THC2 and FPD/AML patients. Furthermore, an increased MYH10 expression is detected in megakaryocytes of FPD/AML patients, but no expression of ANKRD26 is detected in megakaryocytes of THC2 patients.

Then, platelets are obtained from platelet-rich plasma of THC2 and/or FPD/AML patients and ANKRD26 and/or MYH10 level is detected by CLISA and/or Western Blot.

Similarly, an increased ANKRD26 expression is detected in the platelets of THC2 and FPD/AML patients. Furthermore, an increased MYH10 expression is detected in platelets of FPD/AML patients, but no expression of ANKRD26 is detected in platelets of THC2 patients.

**MYH10 and ANKRD26 as markers for diagnosis of thrombocytopenias.**

A double screening of MYH10 and ANKRD26 expression should be thus useful to distinct between these two pathologies presenting similar clinical phenotype; in FPD/AML both MYH10 and ANKRD26 will persist in platelets while in THC2 only ANKRD26 will be detected **(****Figure 3****).**

## Claims

1. An *in vitro* method for diagnosing thrombocytopenia in a subject, said method comprising the step of (i) assessing the *Ankyrin repeat domain-containing protein 26* (ANKRD26) platelet expression in a biological sample from said subject, wherein a ANKRD26 platelet expression is indicative of a thrombocytopenia.

2. The *in vitro* method of claim 1, wherein said thrombocytopenia is *familial thrombocytopenia 2* (THC2) or *familial platelet disorder with propensity for myeloid malignancy* (FPD/AML).

3. The *in vitro* method of any one of claims 1 or 2, wherein said subject is a human.

4. The *in vitro* method of any one of claims 1 to 3, wherein said biological sample is a blood sample.

5. The *in vitro* method of any one of claims 1 to 4, wherein said method is used for distinguishing THC2 and FDP/AML and said method further comprises step of (ii) assessing the MYH10 platelet expression in a biological sample from said subject, and wherein:
a. an ANKRD26 platelet expression and no MYH10 platelet expression is indicative of a THC2, and
b. an ANKRD26 platelet expression and a MYH10 platelet expression is indicative of a FPD/AML.

6. The *in vitro* method of any one of claims 1 to 5, wherein said method further comprises the step of comparing said ANKRD26 and/or MYH10 platelet expression in said biological sample with a control.

7. The *in vitro* method of any one of claims 1 to 6, wherein said method is for testing a subject thought to develop or to be predisposed to developing FPD/AML or THC2.

8. The *in vitro* method of claim 7, wherein said method is for testing a subject thought to be predispose *Acute Myeloid Leukemia* (AML) and/or to *Chronic MyeloMonocytic Leukemia* (CMML).

9. A kit for diagnosing a thrombocytopenia in a subject, which comprises (i) at least one ANKRD26 nucleic acid probe or oligonucleotide or antibody for assessing the ANKRD26 platelet expression in a biological sample from said subject.

10. The kit of claim 9 wherein said ANKRD26 oligonucleotide is at least the PCR primer pair defined by the sequences SEQ ID NO: 5 and SEQ ID NO: 6.

11. The kit of any one of claims 9 and 10, wherein said kit further comprises (ii) at least one MYH10 nucleic acid probe or oligonucleotide or antibody for further assessing the MYH10 platelet expression in a biological sample from said subject.

12. The kit of claim 11 wherein said MYH10 oligonucleotide is at least the PCR primer pair defined by the sequences SEQ ID NO: 7 and SEQ ID NO: 8.

13. The kit of any one of claims 9 to 12, wherein said kit further comprises at least one mean for purifying platelets from the biological sample.

14. The kit of claim 13, wherein said mean is for purifying platelets from a blood sample.

15. Use of a kit as defined in any one of claims 9 to 14 for diagnosing a thrombocytopenia, preferably THC2 or FPD/AML.
